# EUROPEAN PATENT SPECIFICATION

(11) **EP 4 221 512 B1**
(45) Date of publication and mention of the grant of the patent: **26.02.2025**
(21) Application number: 21795211.8
(22) Date of filing: 29.09.2021
(51) Int. Cl.: A23G 1/32, A61K 8/41, A61Q 5/00, A61Q 11/00, G01N 33/68, A01N 37/06, A01N 37/10, A01N 37/36, A61K 8/365, A61K 8/49, A01P 1/00

(54) **COMPOSITIONS, KITS AND METHODS FOR COLLECTING ANALYTE IN A SALIVA SAMPLE**
ZUSAMMENSETZUNGEN, KITS UND VERFAHREN ZUM SAMMELN VON ANALYTEN IN EINER SPEICHELPROBE
COMPOSITIONS, KITS ET MÉTHODES POUR RECUEILLIR UN ANALYTE DANS UN ÉCHANTILLON DE SALIVE

(30) Priority: 29.09.2020 US 202063084897 P
(43) Date of publication of application: 09.08.2023
(73) Proprietor: Decima Diagnostics, LLC, Tampa, FL 33602 (US)
(72) Inventor: GIBBS, Phillip, Atlanta, Georgia 30327 (US)
(74) Representative: Potter Clarkson
(86) International application number: PCT/US2021/052607
(87) International publication number: WO 2022/072467

(56) References cited:
- WO-A2-2020/102808
- US-A- 5 560 906
- US-A- 5 681 549
- US-A1- 2012 263 659
- US-A1- 2019 167 553

## Description

### CROSS-REFERENCE TO RELATED APPLICATIONS

This application claims the benefit of and priority to U.S. Provisional Application No. 63/084,897 filed September 29, 2020.

### FIELD OF THE INVENTION

The invention is generally directed to kits and methods for preserving oral samples containing analyte for subsequent analyte detection.

### BACKGROUND OF THE INVENTION

Saliva is a sample material that is easy to collect without causing stress and can be used for a whole series of analytical purposes. Amongst other things, saliva tests provide valuable information for diagnostic purposes and for controlling the treatment of a number of diseases. A saliva sample allows proteins, hormones, metabolic metabolites, electrolytes and various pharmaceutical substances to be tested.

International patent application WO2020102808A2 is directed to an oral wash for oral fluid collection for subsequent analyte detection using a mass-spectroscopy-based analytical method.

The collection of saliva for purposes of drug testing has several advantages over other matrixes, such as blood and urine given the ease of collection over blood and relevance to current physiological state over urine. These advantages are ideal for cases of confirming a Drug Recognition Experts (DRE) assessment of impairment for Driving Under the Influence of Drug (DUID) arrest with roadside collection of an oral fluid sample for HPLC-MS confirmation. However, in contrast with blood and urine, collected oral fluid is not sterile and contains bacteria, food particulates, and other organic matter than can foster microbial growth post-collection, potentially compromise the integrity of the collected sample.

There remains a need for methods to stabilize oral wash collected saliva samples which include a time lag between sample collection from the oral cavity and the subsequent identification of analyte, using instrumentation such as LC-MS (Liquid chromatography-mass spectrometry), GC-MS( Gas chromatography-mass spectrometry), CE-MS (Capillary electrophoresis-mass spectrometry), HPLC-MS/MS (highperformance liquid chromatography-tandem mass spectrometry), HPLC-TOF, MALDI-TOF (matrix-assisted laser desorption/ionization-time-of-flight) MS, ICP-MS (Inductively coupled plasma mass spectrometry), PCR (Polymerase chain reaction), Real Time (RT)-PCR, Flow Cytometers (FCM), and Automated Analyzer (Immunoassay, biochemical, continuous flow analysis (CFA), etc.)

Therefore, it is the object of the present invention to provide a method for preserving the integrity of analyte present in a collected oral sample, for subsequent analysis.

### SUMMARY OF THE INVENTION

The present invention relates to a kit and a method for collecting a saliva sample, which reduce and preferably, prevent degradation of analytes in the collected saliva sample and/or contamination of the collected sample by subsequent growth of microbes therein, when collected using compositions such as oral wash compositions. The compositions used in the kit of the present invention include an oral wash composition used to collect saliva from a subject, and a post collection stabilization composition which is added to the collected saliva sample.

The oral wash composition includes: (i) one or more salivation agents, (ii) one or more active agents added to maintain oral wash integrity at room temperature, preferably, agents which prevent bacterial/fungal growth, and/or agents, which stabilize the collected oral fluid, (iii) one or more dyes, and (iv) in optional embodiments, an internal tracer. The dye in some embodiments is included as a visual aid indicating the presence of oral wash in a container.

The post collection stabilization composition in some embodiments, includes an acid such as citric acid, and in other preferred embodiments, it is a buffered solution including one or more agents which prevent bacterial/fungal growth, and/or agents, which stabilize the collected oral fluid and one or more dyes. The dye is preferably included as a visual aid in the post collection stabilization composition, distinct from the oral wash dye, indicating that the post collection stabilization composition was added post-collection of the oral wash. In other embodiments, the dye in the post collection stabilization composition can also be a pH indicator dye to show the post-collection oral fluid is in the appropriate pH range for analyte stability.

The kit of the present invention is defined in claim 1.

The disclosed compositions (oral wash and post collection stabilization composition) maintain the integrity of analytes in the collected specimen prior to analysis for presence of analyte in the sample, for example, by substantially reducing or preventing growth of contaminants, such as bacteria, on organic matter typically co-collected from the oral cavity. The low pH and chelating environment can also render certain enzymes, such as ribonuclease (RNase) present in saliva, inactive thus protecting fragile analytes like RNA associated with viral infection. In addition, the low pH environment can also denature proteins/viruses rending them inactive/noninfectious. The disclosed compositions and methods are used to collect a saliva specimen for detection or the presence/quantifying analytes, including but not limited to RNA, DNA, polypeptides, small molecules, etc. Additionally, the disclosed compositions allow the collection and stabilization of analytes such as RNA, at room temperature, uses food safe components, and an easy to use collection method not requiring a medical professional for proper collection. Thus, oral wash/post collection stabilization solutions can be used in kits where the end user collects the samples, at home and forwards to a lab for further analysis.

The analyte in the collected sample can be directly detected subsequently, by a suitable analytical technique, based on the analyte of interest. Exemplary techniques include but are not limited to PCR techniques (RT-PCR), immunoassays, or mass-spec-based analytical methods such as LC-MS, GC-MS, CE-MS, HPLC-MS/MS, HPLC-TOF or MALDI-TOF assay, without loss of analyte as a result of contaminants. In embodiments using a mass-spec-based analytical method such as LC-MS, GC-MS, CE-MS, HPLC-MS/MS, HPLC-TOF or MALDI-TOF assay, the compositions preferably include an internal tracer. In a preferred embodiment, the internal tracer is a stable labelled heavy isotope. In a more preferred embodiment, the internal tracer is caffeine-¹³C₃. Optionally, the internal tracer is a stable heavy isotope-labelled variant of the analyte of interest.

The oral wash and post collection stabilization composition are used to collect a saliva specimen, for subsequent determination of the presence of an analyte of interest, in the specimen. Thus, the dye included in the oral wash composition should be of a color easily distinguishable from the dye in the post collection stabilization solution. In one embodiment, the oral wash includes a yellow dye, and the post collection stabilization solution includes a red dye. The method includes rinsing the oral cavity with the disclosed oral wash for a period of time effective to stimulate saliva production and collecting the resulting fluid (oral wash plus saliva) into a container. The sample is introduced into the container by the subject spitting into the container. In one embodiment, the method preferably does not include using material such as a cotton swab, to collect the saliva mixed with oral wash.

The method of the present invention is defined in claim 11.

The post collection stabilization composition is then introduced into the container containing oral wash plus saliva, and the resulting change in color to color of the dye present in the post collection solution is indicative of the addition of the post collection solution into the specimen container. The saliva sample is then subjected to further analytical techniques, based on the analyte of interest. In one embodiment, the sample is subjected to RT-PCR following RNA extraction, to detect the presence of the SARS-CoV-2 RNA in the sample. In other embodiments, the sample is subjected to an immunoassay, to detect the presence of anti- SARS-CoV-2 IgA, IgG, IgM or combinations thereof. In still other embodiments, the sample is subsequently subjected o to a mass-spec-based analytical method such as LC-MS, GC-MS, CE-MS, HPLC-MS/MS, HPLC-TOF or MALDI-TOF to detect the presence of an analyte in the sample.

Compared to blood-based or urine-based detection and diagnostic methods, the use of oral samples provides an easy approach for sample collection, especially when titrating the dose is important. Urine-based detection and diagnostic methods are not useful for dose titrations. The use of the oral wash is beneficial for sample collection from children. It provides the ease of collection and minimize liability concerns.

### BRIEF DESCRIPTION OF THE FIGURES

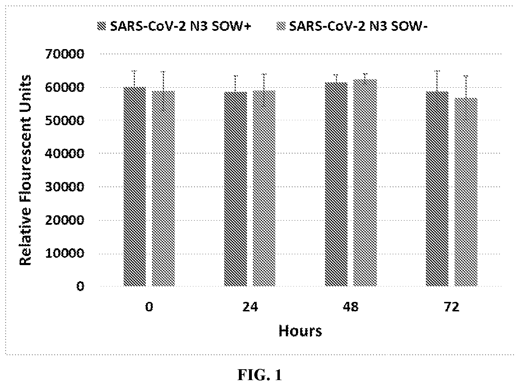

**FIG. 1** is a bar graph of array data showing detection of SARS-CoV-2 N3 target gene relative fluorescent units (RFU) at various time points after spike into SOW+ (left bar at each time point) and SOW-(right bar at each time point. Signals above 10,000 RFU are considered positive.

### DETAILED DESCRIPTION OF THE INVENTION

### I. DEFINITIONS

A "saliva sample" refers to samples derived from saliva from an animal that produces saliva. Saliva is a component of oral fluid produced in most animals.

A "filtered sample" refers to a saliva sample that has been processed to remove cells by separating the cell-phase and the fluid phase of saliva. A filtered sample can have more than 50%, more than 75%, more than 95%, or a 100% removal of cells. A sample is filtered to avoid mechanical rupture of cellular elements that could contribute to the detection of unwanted analytes in the cell-free phase. A filtered sample can further exclude extraneous substances, including but not limited to, food debris. Filtering the sample prior to HPLC-MS analysis is really to remove any particulate matter (food, dirt, bacteria, etc....) that may be collected in the oral fluid from the oral cavity. "Cells", like epithelial cells from the cheeks, are probably small contributors to the components that need to be filtered from the sample. In addition, removal is mainly to prevent these large particulates clogging up the HPLC as opposed to directly interfering with the mass spec analysis since the HPLC column + guard would filter them out anyway.

"Reverse transcription polymerase chain reaction (RT-PCR)" as used herein refers to a laboratory technique combining reverse transcription of RNA into DNA (in this context called complementary DNA or cDNA) and amplification of specific DNA targets using polymerase chain reaction (PCR).

### II. COMPOSITIONS

The disclosed compositions include an oral wash composition for collecting a saliva sample, and a post collection stabilization solution/agent. The post collection stabilization composition can include an acid such as citric acid. In a preferred embodiment however, the saliva in the oral wash is stabilized post collection using a buffered solution, which in some embodiments can serve to minimize dilution, maintains the post collection stabilized wash at a more consistent pH. This can be advantageous for follow on testing procedures like lateral flow assays, which typically need added buffer solutions to the sample, prior to the assay. In this case, a buffered post collection stabilization composition/solution serves to simplify a follow-on test in lateral flow by avoiding the need for an additional buffer additive step (add saliva/blood, then add buffer solution to start assay). The buffering in the oral wash and buffer post-collection should also make the ionic composition more consistent which is also beneficial for follow on test as lateral flows assays tend to require optimization around pH and ionic strength to ensure consistent results. The low pH of the post-collection stabilized oral wash also denatures proteins present in saliva, such as mucins, and contributes to a lower viscosity of the collected oral fluid relative to saliva collected without a salivating agent (sialagogue). The lower viscosity is advantages for the capillary flow conditions of a lateral flow strip and is also easier for liquid handling robots in automated sample prep.

The oral wash preferably includes a sialagogue and one or more additional agents such as a dye, stabilizing agents, etc. As used herein, a sialagogue is a substance that increases the flow rate of saliva. In some embodiments, the oral wash may include an internal tracer, which can be directly detected by a mass-spec-based technique such as LC-MS, GC-MS, CE-MS, HPLC-MS/MS, HPLC-TOF or MALDI-TOF assay, along with all the other analytes being tested in the collected oral fluid.

The method of saliva sample collection using the disclosed oral wash eliminates the need for additional instrumentation (other than the mass-spec-based technique used to detect the analyte) to detect an internal standard in the oral wash, for example, oral washes that use an internal tracer dye which relies on a photometric measurement, and subsequent calculation, for example, to determine the dilution correction factor (U.S. Patent No. 7,883,724). Because the internal tracer included in the disclosed oral wash is selected so that it can be detected by the same instrument used to detect the analyte of interest in the oral sample collected and at the same time as the analyte of interest, additional instrumentation and separate sample prep is not needed when the disclosed oral wash and methods are used for saliva sample collection..

The pH range of the oral wash is between about 3.0 and about 6.2. The pH of the oral wash can be maintained by adding a buffering agent such as citrate. In some embodiments, the pH of the oral wash is adjusted to at or below 4.6 to improve shelf life (inhibit bacterial growth, acidified food stable) and encourage salvation. Having an acidic pH of the oral wash can also ionize the analytes in the form of weak bases, thereby increasing total oral fluid concentrations of the analytes. The post collection stabilization solution is buffered at a pH ranging between 3 and 6, in some preferred embodiments, at an acidic pH, for example, between 3 and 4. Having a higher molarity and lower pH composition of the smaller volume of stabilizer, targets the collected solution plus stabilizer having a final pH under 4.6 to prevent microbial growth and ideally a pH of 4.0 for maximal stability of free RNA that may be released by the resulting denaturation/de-enveloping of any viral capsids from associated infections.

The post collection stabilization composition preferably includes a dye and one or more active agents added to maintain oral wash integrity at room temperature, preferably, agents, which prevent bacterial/fungal growth, and/or agents, which stabilize the collected oral fluid. The dye in the post collection stabilization composition is typically of a different color from the dye included in the oral wash. The dye serves as a visual confirmation when the post collection stabilization solution is added to the vial containing the oral wash, post saliva collection. In some preferred embodiments, the dye included in the post collection stabilization composition is a pH indicator dye. The pH indicator dyes are used to visually verify the post collection stabilization composition has been added and the pH is below a desired cutoff, for example, 4.5, when the sample is received in the lab. Other examples of dyes that have transitions in the right include Bromophenol blue and Bromocresol green region (3.0-4.6 and 3.8-5.6 respectively) or Methyl Orange and Methyl Red (3.0-4.4 and 4.4-6.2). One can readily select a suitable dye for use as a color indicator as disclosed for example, in Kahlert, et al., "Colour maps of acid-base titrations with colour indicators: how to choose the appropriate indicator and how to estimate the systematic titration errors" doi:10.1007/s40828-016-0026-4.

Post collection stabilization composition is used herein interchangeably with Post collection stabilization fluid.

### a. Internal Tracer

The internal tracer is a stable labelled heavy isotope. The internal tracer can facilitate dilution correction (normalization), validity conformation (preventing false negative results), and/or mitigate adsorptive losses.

Advantages of using a stable heavy isotope-labelled internal tracer is that such internal tracers cannot contribute/interfere with the signal at the most abundant monoisotopic mass. Preferred tracers are stable labelled heavy isotopes of commercially available analytes that can easily be detected and distinguished from potential background interferants.

Stable labelled isotopes are preferable over deuterated analytes due to problems of hydrogen exchange upon long-term storage in the oral wash solution (Davison, et al., Annals of Clin Biochem., 560:274 (2013)). C¹³ and N¹⁵ stable labelled isotopes are most preferred and are commercially available.

In a more preferred embodiment, the internal tracer is heavy-isotope labeled caffeine, such as caffeine-¹³C, caffeine-¹³C₃, and caffeine-¹³C,D₃. Caffeine is ubiquitous in the population, works well with the HPLC-MS method (sharp peaks, great S/N), and has many options for heavy labelled isotopes commercially available.

Caffeine-¹³C₃ was found to be a readily available heavy labelled isotope of a common analyte that presents no danger when included in the oral wash at low concentrations (100 ng/ml) for example, between 100 and 1000 ng/ml. In addition, having the three C13's places the analyte sufficiently far enough away (+3 Da) from the native analyte as to avoid background interference from endogenous caffeine that can be present in high levels for frequent caffeine users. The presence of a background can be addressed by quantitatively measuring endogenous caffeine collected in the oral fluid and applying a correction to the caffeine-¹³C₃ value used to determine the dilution factor correction. Caffeine-¹³C,D₃- also provides a large shift in the m/z to distinguish from endogenous caffeine. This additional internal standard/tracer can be added to the oral wash at the same concentration range as that of caffeine-¹³C₃. In a preferred embodiment, this internal tracer (Caffeine-¹³C,D₃), is utilized in the lab sample prep of the oral fluid for HPLC-MS/MS analysis. The addition of the internal standard to samples in the lab prep is utilized to normalize ion suppression/enhancement in the source to improve quantitative analysis of the associated analytes. Of note, the internal tracer as used herein (is distinct from the internal standard) is used to determine the dilution in the preanalytical step, if needed.

Caffeine also has stable labelled plus deuterated versions of the analyte that allow post collection addition of an internal standard to correct both measurements caffeine and caffeine-¹³C₃ for ion enhancement/suppression due to matrix effects. In some embodiments, the internal standard is the [13C, 2H3]-caffeine, caffeine-13C-D3 deuterated standard, and/or single C13 stable labelled caffeine. This is separate and distinguishable from the stable labeledcaffeine-¹³C₃.

In some embodiments, the internal tracer is a stable labelled heavy isotope of the analyte of interest. For example, when the analyte is a smallmolecule drug such as THC, methadone, EDDP (2-ethylidene-1,5-dimethyl-3,3-diphetiylpyrrolidine), aripiprazole and metabolite (e.g., dehydroaripiprazole or OPC-3373), the internal tracer can be a heavy isotope-labeled variant of the analyte. The heave isotope labeling can be one or more of the carbon and/or nitrogen atoms of the analyte, and/or on one or more of the hydrogen atoms of the analyte, as described above.

Optionally, the oral wash does not contain any internal tracer. For example, analytes with a high diffusion constant in water, such as alcohol, can quickly reach distribution equilibrium after the oral wash is in contact with the oral cavity. Such analytes may not need the use of any internal tracer in the oral wash for dilution correction or validity confirmation.

### b. Salivation Agents/Sialagogues

The oral wash can also include agents to induce salivation. Examples include but are not limited to inorganic and/or organic edible acids and/or salts or mixtures thereof, for example, phosphoric acid, lactic acid, citric acid, and ascorbic acid. Examples of salivary stimulants are disclosed for example in U.S. Patent No. 4,820,506. Compositions useful within the context of xerostomia may also be used. For example, nutritionally acceptable and chemically defined compounds may be administered as described in U.S. Pat. App. No. 2007/0128284 where a sulfur-containing antioxidant such as N-acetylcysteine is combined with a polymeric base, or in U.S. Pat. App. No. 2004/0076695 where omega-3 fatty acids are used, in various compositions. In yet further known compositions, peroxidized lipids (typically plant oils) and silica are used to alleviate xerostomia as taught in U.S. Pat. App. No. 2006/0078620. In yet other known methods, glycerol may be employed to improve dry mouth conditions as noted in U.S. Pat. App. No. 2009/0263467. U.S. Patent No. 9, 5972,287 discloses compositions, which employ one or more plant pulp products and/or a proanthocyanidin to stimulate saliva and reduce dry mouth.

The concentration of the substance with saliva stimulating properties is selected so as to be between 0.0005% and 10% of the oral wash composition, preferably between 0.01%, and 5% and more preferably, between 1%, and 2%.

### c. Active Agents

The compositions include one or more active agents to maintain oral wash integrity at room temperature, preferably, agents, which prevent bacterial/fungal growth, and/or agents, which stabilize the collected oral fluid. Examples include, but are not limited to, food-grade conservatives such as potassium benzoate, trisodium citrate dihydrate, citric Acid monohydrate, disodium EDTA, sodium Benzoate and Potassium sorbate:

Saliva contains several bacterial proteases, which can degrade salivary proteins affecting some techniques. To avoid this pre-analytical issue, it is useful to include protease inhibitors and stabilizing substances (such as aprotinin, leupeptin, antipain, pepstatin A, phenyl methyl sulfonyl fluoride, EDTA, thimerosal).

A particularly preferred simple solution to the concerns presented by microbes present in a collected saliva sample, is to add a post collection agent that stabilizes the sample inhibiting microbial growth. Ideally this agent is inexpensive, easily added in the field, and is compatible with the subsequent analytical method, for example, HPLC-MS analysis without requiring additional processing to remove the stabilization agent. One preferred agent/ method to address these concerns can be the addition of between 50-150 mg of citric acid, more preferably ~100 mg of citric acid powder directly to the collected oral fluid. Packets of citric acid powder are readily available, inexpensive, easily added, and drop the pH to well under pH 4.0 which is effective to prevent microbial growth. In addition, since citrate buffer is already part of the preferred oral wash formulation, no additional sample processing is required. Other additives post collection can also be utilized and fulfill the same desired characteristics (acetic acid, antibiotics, chlorhexidine, etc.) but given the fact that citrate buffer is already utilized in the oral wash collection buffer, the addition of citric acid directly to the collected oral fluid is a preferred stabilization solution to minimize dilution of the collected sample. The preservative(s) can be added into the oral wash as a solution. Preferably, a combination of agents are used, for example, Trisodium Citrate dihydrate, Citric Acid monohydrate, Disodium EDTA, Sodium Benzoate and Potassium sorbate: An additional benefit of adding the stabilizer components as a solution, is the ease product packaging for manufacture (stabilizer packets/pouches) and easier to handle/dispense into the collected oral fluid. Still another benefit of adding the post-collection stabilizer is the collected sample is already primed for laboratory procedures known to improve RNA isolation/detection (attached reference) In this case high salt can be added to further aid in precipitation of proteins and DNA leaving the RNA in solution. This aids the further purification and/or concentration by traditional RNA isolation methods such as but not limited to magnetic beads, silica spin columns, or co-precipitants such as glycogen/glycogen derivatives by removing unwanted contaminants that would compete for binding or precipitate in the with the co-precipitated RNA reducing the effectiveness of this isolation/concentration step. The enhanced RNA isolation afforded by the post collection stabilizer addition may also remove or reduce the need for a centrifugation of the initial collected sample for prep into molecular testing thus aiding/streamlining sample prep in testing scale up

### d. Additional Components

Additional components that can be included in the oral wash/post collection stabilization composition include, but at not limited to are FDA approved food dyes (these are also synthetic dyes and essentially homogenous, one component, in contrast to natural sources which are less ideal due to heterogenous nature of the mixes) and flavoring agents. Allura Red AC was found to be a readily available dye that could be incorporated in the wash and detected on the mass-spec-based method used to detect analytes of interest. In some preferred embodiments, the compositions do not include Allura Red AC as an internal tracer.

Therefore, in one preferred embodiment, the oral wash includes tartrazine (yellow in color), and the post collection stabilization composition includes Allura Red AC. The tartrazine serves as a visual aid to the user indicating the presence of the oral wash in its vial, and the red color in the collected sample serves as a visual confirmation that the post collection stabilization solution was added to the collected saliva sample. Alternative yellow dyes that can substitute tartrazine (Yellow 5) include sunset yellow (Yellow 6) and quinoline yellow (Yellow 10). Alternative red dyes include ponceau 4R (Red 10), camoisunbe (red 4) erythrosine (Red 3).

An additional benefit of tartrazine and Allura Red AC is that these dyes have been shown not to interfere with typical RT-PCR test. The Allura Red AC dye has also been observed to bind to food proteins and, based on observations of collected oral fluid specimens, potentially aid in flocculation/precipitation of saliva proteins denatured by the low pH chelating environment after the stabilizer is added. This further reduces the viscosity of the collected sample and clarifies the oral fluid for further analysis potentially avoiding the need for additional processing steps such as centrifugation.

However, other dye combinations can be selected for use in the oral wash/post collection stabilization fluid, so long as the color of the dye selected for the oral wash is different in color from the dye selected for the dye included in post collection stabilization fluid, such that the color change following addition of the post collection stabilization fluid into the oral wash is readily apparent by visual inspection. Example of dyes that can be used in the oral wash composition/ post collection stabilization solution include, but are not limited to, riboflavin, riboflavin-5'-phosphate, chlorophylls and chlorophyllines, copper-containing complexes of chlorophyll and Chlorophyllines, caramel dye, simple sugar-based dye, sulphite lye-caramel die, ammonia-caramel dye, ammonium sulphite-caramel die, vegetable carbons, paprika extract, capsanthin, capsorubin, beetroot, betanine, anthocyans, iron oxides and hydroxides, azorubin, carmoisin, Ponceau 4R, cochineal red A, , patent blue V, indigotin, indigo carmine, brilliant blue FCF, green S, fast green, brilliant black BN, black PN, brown HT, lycopene, beta-apo-8'-carotinal (C30), beta-apo-8'-carotinic acid (C30)-ethyl ester, lutein, substances of Maillard compounds, tartrazine, curcumin, saffron, quinoline yellow, sunset yellow FC/ yellow orange S, orange B, citrus red, cochineal, carminic acid, carmine, and carotene. In some preferred embodiments, the oral wash solution does not include a dye such as tartrazine, curcumin, saffron, quinoline yellow, sunset yellow FCF/yellow orange S, cochineal, carminic acid, carmine, or carotene.

The concentration of the dye in the disclosed oral wash can be between 0.0001% and 5% of the oral wash, preferably between 0.005% and 1%.

The oral wash can also include flavoring agents or flavor enhancers to improve the flavor of the wash. Useful flavoring agents can include a sugar and/or sugar substitute, for example, saccharose, maltose, fructose, or sweeteners such as for example saccharin, aspartame, and/or mixtures thereof. Any orally acceptable natural or synthetic flavorant can be used, including without limitation vanillin, sage, marjoram, parsley oil, spearmint oil, cinnamon oil, oil of wintergreen (methyl salicylate), peppermint oil, clove oil, bay oil, anise oil, eucalyptus oil, citrus oils, fruit oils and essences including those derived from lemon, orange, lime, grapefruit, apricot, banana, grape, apple, strawberry, cherry, pineapple, etc., bean- and nutderived flavors such as coffee, cocoa, cola, peanut, almond, etc., adsorbed and encapsulated flavorants and the like. Also encompassed within flavorants herein are ingredients that provide fragrance and/or other sensory effect in the mouth, including cooling or warming effects. Such ingredients illustratively include menthol, menthyl acetate, menthyl lactate, camphor, eucalyptus oil, eucalyptol, anethole, eugenol, cassia, oxanone, a-irisone, propenyl guaiethol, thymol, linalool, benzaldehyde, cinnamaldehyde, N-ethyl-p-menthan-3-carboxamine, N,2,3-trimethyl-2-isopropylbutanamide, 3-(1-menthoxy)-propane-1,2-diol, cinnamaldehyde glycerol acetal (CGA), menthone glycerol acetal (MGA) and the like. In certain embodiments, the composition optionally further comprises at least one sweetener, useful for example to enhance taste of the composition. Any orally acceptable natural or artificial sweetener can be used, including without limitation dextrose, sucrose, maltose, dextrin, dried invert sugar, mannose, xylose, ribose, fructose, levulose, galactose, sucralose, corn syrup (including high fructose corn syrup and corn syrup solids), partially hydrolyzed starch, hydrogenated starch hydrolysate, sorbitol, mannitol, xylitol, maltitol, isomalt, aspartame, neotame, saccharin and salts thereof, dipeptide-based intense sweeteners, cyclamates and the like. One or more sweeteners are optionally present in a total amount depending strongly on the particular sweetener(s) selected, but typically 0.01% to 15% by weight of the composition, or 0.1% to 10% by weight. In a preferred embodiment, sucralose is used as the sweetener due to the high sweetness relative to sugar (400-700 times sweeter), inability to support microbial/fungal growth, and the compound is heat stable.

Optionally, the oral wash contains a fluorescence dye rather than a colorimetric dye. The fluorescence dye can be used as an internal tracer for dilution correction. In preferred embodiments, the fluorescence dye does not inference with mass-spec analysis of the analyte of interest in the oral sample.

Optionally, the oral wash contains one or more proteins to minimize the loss of analyte due to surface absorption during oral sample collection, transportation, storage, and/or analysis. Preferably, the proteins are from a non-human source. Exemplary proteins include, but are not limited to, ovalbumin, albumin, and lysozyme (e.g., chicken lysozyme).

Optionally, the oral wash contains one or more mass-spec compatible detergents and/or emulsifiers. Exemplary mass-spec compatible detergents and emulsifiers include, but are not limited to, sodium 3-(4-(1,1-bis(hexyloxy)ethyl)pyridinium-1-yl)propane-1-sulfonate (PPS SILENT^{®} surfactant), INVITROSOL^{®}, sodium 3-((1-(furan-2-yl)undecyloxy)carbonylamino)propane-1-sulfonate (PROTESEMAX^{®} surfactant), and sodium 3-[(2-methyl-2-undecyl-1,3-dioxolan-4-yl)methoxy]-1-propanesulfonate (RAPIGEST^{®} surfactant).

The oral wash is preferably sterilized using methods known from the prior art, for example, sterile filtration or autoclaving.

An exemplary Oral wash composition is provided herein.

### 1. Oral Wash (1 L, 50 mM, pH 4.2)

| | |
|---|---|
| Trisodium Citrate dihydrate (mw: 294.1 g/mol): | 6.75 g |
| Citric Acid monohydrate (mw: 210.14 g/mol): | 5.65 g |
| Tartrazine: | 5 mg |
| Sucralose: | 100 mg |
| Disodium EDTA: | 300 mg |
| Sodium Benzoate: | 500 mg |
| Potassium sorbate: | 500 mg |
| Butyl 2-methylbutyrate: | 6 µL |
| Methyl isobutyrate: | 6 µL |
| Caffeine-13C3 | 20 µg |

A preferred preparation scheme is as follows. Trisodium Citrate dihydrate is added to water and mixed until dissolved. Disodium EDTA is added and mixed until dissolved. Sodium Benzoate, Potassium sorbate, and Sucralose are added, and mixed until dissolved. Add Citric Acid monohydrate and mix until dissolved. A pH (4.2±0.1) check is conducted; and as needed, adjusted with food grade Citric Acid or NaOH (concentrated solutions preferred). Final volume is brought to 1 L. Heat sterilize or sterile filter solution. After cooled to room temp, where applicable, add Butyl 2-methylbutyrate, Methyl isobutyrate, and 50 µL Tartrazine (FD&C Yellow 5, 100 g/L stock in H₂Ostock).

An exemplary post collection stabilization solution is provided herein

### 2 Post Stabilization solution [1 L, (1 M citrate buffer), pH 3.7]

| | |
|---|---|
| Trisodium Citrate dihydrate (mw: 294.1 g/mol): | 100.49 g |
| Citric Acid monohydrate (mw: 210.14 g/mol): | 138.10 g |
| Disodium EDTA: | 1000 mg |
| Sodium Benzoate: | 1000 mg |
| Potassium sorbate: | 1000 mg |
| Allura Red AC (FD&C Red 40): | 100 mg |

A preferred preparation scheme is as follows:
Blending order: Trisodium Citrate dihydrate is added to water and mixed until dissolved; Disodium EDTA is added and mixed until dissolved; Sodium Benzoate, Potassium sorbate, Sucralose are added, and mixed until dissolved; Citric Acid monohydrate is added and mixed until dissolved. pH is checked (pH (3.7±0.1)), and as needed, adjusted with food grade Citric Acid or NaOH (concentrated solutions preferred); the final volume is brought to 1 L; Heat sterilize or sterile filter solution. After cooled to room temp, where applicable, add 1000 µL Allura Red AC (FD&C Red 40, 100 g/L in H₂O stock).

### III. KITS

Kits for collecting saliva samples from a subject. The kits include an oral wash composition as disclosed herein, and a post collection stabilization solution. The oral wash solution is in a collection tube and the post collection stabilization solution is provided in a separate container, for example, a syringe, tearable pouch or screw cap container. The oral wash container preferably includes a sufficient volume of oral wash, for saliva collection, for example, between 1-3ml of oral wash, preferably, about 2 ml. The post collection stabilization solution can be supplied as a 100 µl to 1 ml solution, preferably, as a 300-800 µL solution, and more preferably as a 500 µl solution. In one preferred embodiment, the kit includes of a clear zip-closure bag containing a screw-cap conical specimen tube holding 2ml oral wash composition and a separate pre-filled ampule containing 0.5ml of stabilizing solution.

### IV. METHODS OF USING

The disclosed oral wash is used to collect saliva from the oral cavity, and the collected sample is subsequently subjected to analysis using analytical techniques depending on the analyte of interest, in order to determine the presence of an analyte in the saliva. A preferred technique is a mass-spec-based technique such as LC-MS, GC-MS, CE-MS, HPLC-MS/MS or HPLC-TOF, in order to determine.

The disclosed method eliminates the need for additional instrumentation (other than mass spectrometry) to detect the internal tracer in the oral wash. In contrast, oral washes that rely on an internal tracer dye requires photometric measurement (using a spectrophotometer, for example) and subsequent calculation, for example, to determine the dilution correction factor. Therefore, in preferred embodiments, the disclosed method does not include the step of photometric measurement.

Analytes that can be measured in a saliva sample include, but are not limited to calcium, magnesium cortisol, alcohol, drugs and drug metabolites, biomarkers for cancer, analytes for medication monitoring, viral RNA, for example, influenza virus, Severe acute respiratory syndrome (SARS) coronavirus, SARS coronavirus 2 (SARS-CoV-2), the strain of coronavirus that causes coronavirus disease 2019 (COVID-19) or RNA, and microbial RNA or DNA. Additional analytes are listed in Table 1.

In some embodiments, the oral wash contains a heavy isotope-labeled variant of the analyte as an internal tracer. In some embodiments, the oral wash contains heavy isotope-labeled caffeine as an internal tracer. In some embodiments, the oral wash does not contain any internal tracer.

**Table 1. Analytes detected in saliva**

| **Analyte** | **Example** |
|---|---|
| **Hormones** | |
| **Steroids** | Cortisol, androgens (testosterone), estriol, estrogen, progesterone, aldosterone, DHEAS |
| Antibodies | IgG, IgA, sIgA, IgM |
| Growth factors | EGF, NGF, VEGF, IGF |
| Cytokines and Chemokines | IL-1 beta, IL-8, IL-6, MCP-1, CxeCL1, GRO-1 alpha, troponin 1, TNF alpha |
| Nucleic acids | Human DNA, microbial DNA, mRNA, siRNA, micro RNA (miR-125a and miR-200a) |
| Proteins | 100's-1,000s (antiepileptic's, immuno-suppressant, anti-cancer) |
| Drugs | Drugs or abuse (NIDA 5), ethanol, therapeutic drugs, anticonvulsants, antipyretic/analgesics, anti-bacterial agents, bronchodilators, cotinine |

The disclosed oral wash is useful to test drugs of abuse in clinical, workplace, driving under the influence of drugs (DUID), drug treatment, and criminal justice settings. The main advantages of oral fluid collection are the simplicity and noninvasiveness of sample collection, which can be easily observed, obviating the need for special restroom facilities and same-sex collectors and making adulteration more difficult. In the U.S., oral fluid (OF) testing is expanding at a rapid pace in nonregulated workplace testing, treatment, and driving under the influence of drugs (DUID) programs. In 2004, Substance Abuse and Mental Health Services Administration (SAMHSA) proposed recommended guidelines (Table 2) for mandated federal workplace OF testing.

**TABLE 2. SAMHSA, DRUID, and Talloires recommended oral fluid cutoffs.))**

| Drug/analyte | SAMHSA screen, µg/L | SAMHSA confirmation, µg/L | DRUID confirmation, µg/L | Talloires confirmation, µg/L |
|---|---|---|---|---|
| Cannabinoids | 4 | 2^{b} | 1^{b} | 2 |
| Opiates | 40 | | | |
| Morphine | | 40 | 20 | 20 |
| Codeine | | 40 | 20 | 20 |
| 6AM | 4 | 4 | 5 | 5 |
| Methadone | - | | 20 | 20 |
| Phencyclidine | 10 | 10 | - | - |
| Amphetamines | 50 | | | |
| Amphetamine | | 50 | 25 | 20 |
| Methamphetamine | | 50^{c} | 25 | 20 |
| MDMA | 50 | 50 | 25 | 20 |
| MDA | | 50 | 25 | 20 |
| MDEA | | 50 | 25 | 20 |
| Cocaine or benzoylecgonine | 20 | 8 | 10 | 10 |
| Benzodiazepines | - | | | |
| Flunitrazepam | | - | 1 | - |
| Diazepam | | - | 5 | - |
| Alprazolam | | - | 1 | - |
| Oxazepam | | - | 5 | - |
| Nordiazepam | | | 1 | - |
| Lorazepam | | | 1 | - |
| Clonazepam | | | 1 | - |

| | | | | |
|---|---|---|---|---|
| ^{a} (reproduced from Bosker et al., *Clin. Chem.,* 55(11):1910-1931 (2009 Driving under the Influence of Drugs, Alcohol and Medicines (DRUID) ^{a}See references: SAMHSA [Department of Health and Human Services, SAMHSA; DRUID ^{b}THC. ^{c}Specimen must also contain amphetamine ≥ method limit of detection. | | | | |

The disclosed methods provide the advantage of using the same instrument (e.g., LC-MS, GC-MS, CE-MS, HPLC-MS/MS, HPLC-TOF or MALDI-TOF) for detecting the analyte of interest and the internal tracer. The use of an internal tracer detectable by the same instrument used to identify analytes in an oral sample solves the problem of normalizing for dilution of the collected oral fluid (internal standard normalization) but does not require a separate method to quantitate the internal tracer. In other words, quantitation of the internal tracer allows one to back-calculate the amount of oral fluid that was collected given that the starting volume of oral wash and starting concentration of the internal tracer are known.

Another aspect of the current oral wash-lab workflow combination is the use of filter vials for minimal sample prep prior to analysis. Since there are no components of the oral wash that are particularly problematic for the LC-MS, GC-MS, CE-MS, HPLC-MS/MS, HPLC-TOF or MALDI-TOF analysis (mostly salts that elute prior to analysis, go to waste via switching valves), a simple in vial filtration can be performed (Thomson filter vials) after addition of internal tracers (normally in MeOH). This greatly reduces the time and expense required for sample prep when compared to the Solid Phase Extraction or Liquid/Liquid extraction required for other methods to remove dyes and surfactants. The disclosed methods therefore afford the advantage of not requiring Solid Phase Extraction or Liquid/Liquid extraction.

Saliva is produced exclusively by three large and numerous smaller saliva glands and is predominantly generated in the oral cavity. The whole fluid present in the oral cavity originates mainly from three salivary glands: parotid, submandibular and sublingual. Minor salivary glands (buccal, labial, palatal, palatoglossal, lingual) located in the oral cavity, gingival crevicular fluid with bacteria, epithelial cells, erythrocytes, leukocytes and food debris can contribute in small volume to the formation of what is designated as "oral fluid" or "whole saliva".

The method includes: (a) rinsing the oral cavity with the disclosed oral wash for a period of time effective to stimulate saliva production, (b) collecting the resulting fluid (oral wash plus saliva) into a container; (c) adding the post collection stabilization fluid to the collected saliva sample, and (d), subsequently subjecting the resulting fluid to a subsequent analytical technique to detect the presence of an analyte in the sample. Thus, in one embodiment, the patient or subject dispenses oral wash into their mouth and swishes an effective amount of time to stimulate saliva production, for example, 30 to 90 seconds, preferably, 60 seconds; the patient then empties the contents of their mouth back into the tube which originally contained the oral wash; premeasured stabilization fluid is added to the collected saliva; specimen is capped and inverted approximately 2-3 ties for mixing. The oral wash works by rinsing the oral cavity and collecting any analyte such as SARS-CoV-2 viral material, present. One collection typically yields 3-7 mL total oral fluid (oral rinse solution plus saliva) after rinsing the oral cavity for 1 minute. The post collection stabilizing solution (0.5mL) is supplied as a part of the collection device in a pre-filled ampule and is added to the oral rinse fluid post-collection by the specimen collector. The stabilization fluid is preferably red therefore once added to the collected sample the oral rinse fluid changes from yellow to pink/red indicating the post collection stabilization step has been performed. The red color also helps reinforce which solution is to be swished for sample collection.

Exemplary techniques, include, but are not limited to a mass spectrometry instrumentation such as LC-MS, GC-MS, CE-MS, HPLC-MS/MS, HPLC-TOF or MALDI-TOF, RT-PCR, lateral flow immunoassays, etc., known in the art Cazacu et 1., J. Clin Microbiol., 42(8):3661-3664 (2004); Thao, et al., Anal. Chem. 2017, 89, 6781-6786 (2017). Cleaning mouth with water (preferably distilled) prior to the oral rinse is preferred, to eliminate residues that may hamper analyses.

The volume of the oral wash composition used for rinsing the oral cavity can be between 0.5 ml and 10 ml, for example, between 1 ml and 7ml, for example, 1 ml. 2ml. 3ml, 4ml, 5ml, 6ml or 7 ml. The oral wash is left in the oral cavity for a period of time effective to stimulate saliva production. The time can be 20 sec-10 mins, for example, 30 sec, 45 sec, 1 min, 2 min, 3min, 4 min, etc. It is particularly preferred to leave the oral wash in the oral cavity for a period of nor more than 1 minute.

The post collection stabilization solution is added to the resultant mixture of oral wash plus saliva, which is subsequently subjected to further analytical techniques such as a mass spectrometry instrumentation such as LC-MS, GC-MS, CE-MS, HPLC-MS/MS, HPLC-TOF or MALDI-TOF, RT-PCR, immunoassays, etc., in order to detect the analyte in the collected saliva. Preferably, collected saliva samples should be refrigerated at 4 °C for processing within 24 to 48 hours after collection. However, the low pH acid stable conditions enable to the sample to be processed after extended periods (≥5 days) at room temperature with no indication of analyte loss for relatively fragile analytes such as naked genomic RNA from covid-19 (ATCC VR-1986D).

In some preferred embodiments, the saliva sample is filtered to provide a saliva sample free of cells and removal of any "particulate matter". Centrifugation can also be used for this purpose. The phrase "free of cells" refers to a sample solution that has been filtered in accordance with the methods of the present invention such that the sample solution is completely or substantially cell-free. Exemplary filters can include, but are not limited to, cellulose fiber matrix, hydrophilic filters, such as those based on polyvinylidene fluoride membrane, or filters based on polypropylene membrane. Filters can have micropores that are a wide variety of sizes, including, but not limited to, 0.22 µm, 0.45 µm and 5.0 µm. The term "filtering" refers to the application of a liquid sample containing cells, e.g. a saliva sample, to a membrane filter. Filtering is the process of removing cells and/or parts of cells from excess fluid in a liquid sample by passing the sample through a microporous membrane filter.

Embodiments that use an internal tracer that is detectable by a mass-spec-based technique such as LC-MS, GC-MS, CE-MS, HPLC-MS/MS, HPLC-TOF or MALDI-TOF, eliminate the need for extensive prep of the samples prior to analysis using these techniques. The sample is loaded I the filter vial according to manufactures instructions (450 µL max, for example, 335 µL collect oral fluid + 100 µL of MeOH with internal standards (typically deuterated since exchange isn't an issue in the MeOH and the time frame post sample prep is too short for this to be of concern) for the HPLC-MS/MS analysis. The ratio is important to keep the prepped samples MeOH content low enough so as to not impact subsequent chromatography (~25% in this case with a 5 uL injection, prepped sample has ~22%). The top part of the filter vial is pressed in the filter the sample, and this is then loaded onto the Mass spec for analysis. This is much easier and cheaper than the sample prep required from other oral collection kits like Quantisal where Solid Phase or Liquid/Liquid extraction must be utilized to clean up and/or concentrate the sample prior to analysis. Otherwise a dilute and shoot method will "dirty" the mass spec very quickly and require frequent mass spec cleaning.

An additional benefit of the lower viscosity of the post-stabilized oral fluid relative to raw saliva is easier filtration during the sample prep. This reduction in viscosity of the sample also aids other diagnostic test such as lateral flow immunoassays strips and is easier for liquid handling robots to pipette accurately. Other additives, such as Dithiothreitol (DTT) or N-acetylcysteine (NAC), are known to lower the viscosity of collected saliva or mucin rich sputum samples by the reduction of disulfide bonds. Therefore, these types of reducing agents are also potential additives to the Post collection stabilization composition to reduce viscosity. However, since the combination of the low pH and chelating environment in the oral wash + stabilizer is already effective in reducing the viscosity of the sample and these reducing agents are subject to oxidative degradation, preferred embodiments rely on the low pH and chelation features of the citrate and EDTA salts for maximizing shelf life of the oral kit components.

A preferred the internal tracer is caffeine-¹³C₃. In embodiments where extremely high levels of endogenous caffeine could potentially contribute to detected internal standard levels, a background correction can easily be applied by quantifying the caffeine levels in the collected oral fluids. A benefit of using caffeine as the internal tracer is the availability of the standard in several heavy label and deuterated versions and non-toxic nature of the analyte compatible with food safe formulations.

An additional feature of this approach to simplify the methods application in the lab has the Laboratory Information Management System (LIMS) implement the dilution calculation directly in the software that generates the analyte reports from the LC-MS, GC-MS, CE-MS, HPLC-MS/MS, HPLC-TOF or MALDI-TOF data. This algorithm can make any necessary caffeine background correction on a per sample basis, determine the undiluted internal tracer level from QCs run with the batch, and use the calculated dilution factor to correct reported concentration levels. This automates the calculation process and simplifies the workflow from the standpoint of the lab technician.

Collected samples can be analyzed for analyte shortly after collection or the sample can be stored at -80°C, -20°C, or 4°C. Collected samples can be stored at room temperature and are preferably assayed for the presence of analyte on the same day, or shortly thereafter. In some embodiments, the collected sample can be assayed for the presence of analyte up to 28 days post collection.

### A. Viral Infection diagnostics

Saliva samples, collected as disclosed herein can be used for viral infection diagnostics, for example, to detect the presence of viral RNA or antibodies against a virus (indicating exposure to the virus). In one embodiment, the virus is a respiratory virus. Respiratory viruses are the most frequent causative agents of disease in humans, with significant impact on morbidity and mortality worldwide. Common respiratory agents from several virus families are well adapted to efficient person-to-person transmission and circulate in a global scale, and community-based studies conducted over the past five decades or so confirm that these viruses are the predominant etiological agents of acute respiratory infections (ARIs). The respiratory viruses that most commonly circulate in all continents as endemic or epidemic agents are influenza virus, respiratory syncytial virus, parainfluenza viruses, metapneumovirus, rhinovirus, coronaviruses, adenoviruses, and bocaviruses. One preferred viral infection is a CARS-CoV-2 infection.
Common respiratory viruses, their classification, principal syndromes, and main detection methods (Boricristiani et al., Respiratory Viruses. Encyclopedia of Microbiology. 2009;500-518. doi: 10.1016/B978-012373944-5.00314-X)

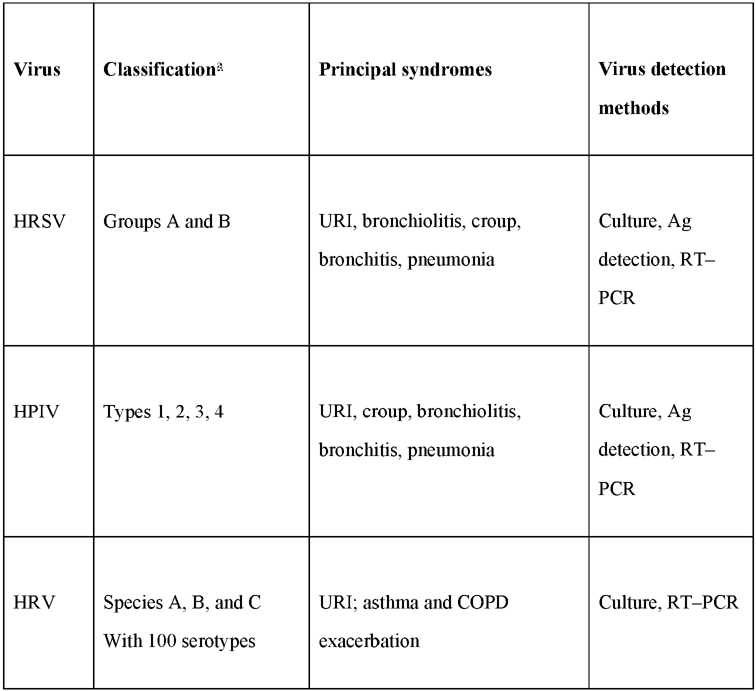

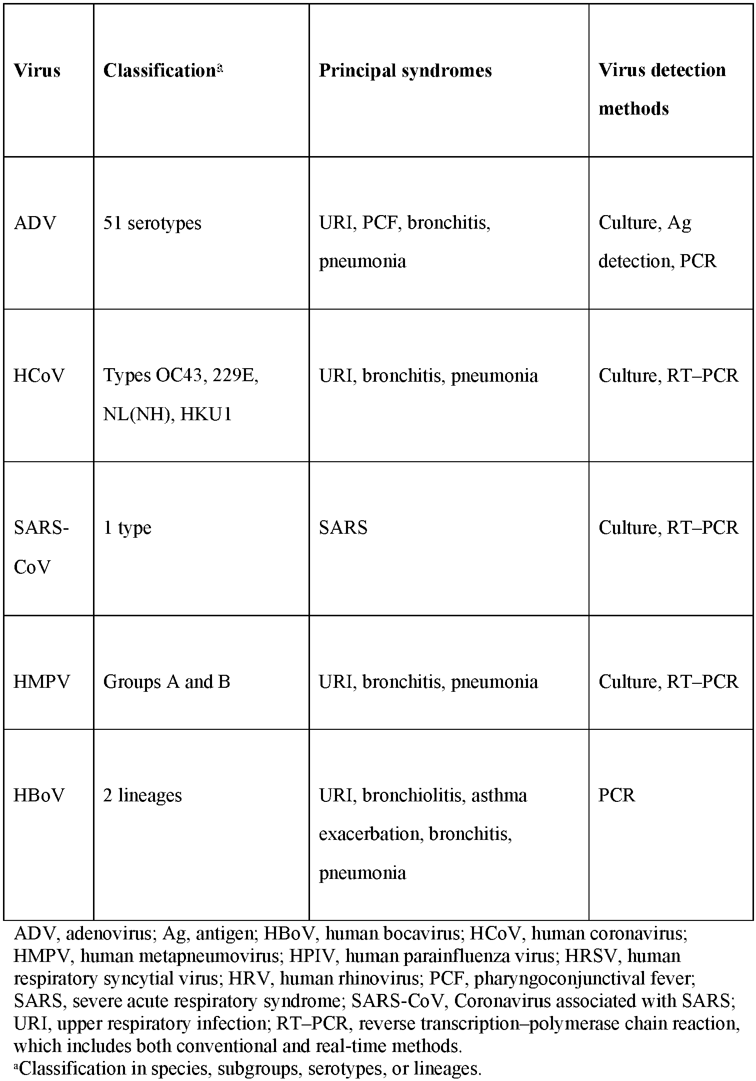

Coronaviruses (CoV) are a large family of viruses that cause illness ranging from the common cold to more severe diseases such as Severe Acute Respiratory Syndrome (SARS). Coronaviruses are zoonotic, meaning they are transmitted between animals and people. Several known coronaviruses are circulating in animals that have not yet infected humans. SARS-CoV-2 is a new strain that had not been previously identified in humans.

RNA can be extracted from saliva samples collected as disclosed herein, using methods known in the art, and subsequently subjected to known techniques for detecting the presence of the relevant RNA such as SARS-CoV-2, in the sample. RNA molecules can also undergo strand scission when heated in the presence of divalent cations, such as Mg2+ or Ca2+, at >80°C for 5 minutes or more. Thus, a chelating agent should be present whenever there is a requirement for heating RNA. RNA Storage Solutions are expressly designed for storing RNA. These solutions typically contains1 mM sodium citrate, which is an efficient chelator of divalent cations, and has a relatively low pH (~6.4) that minimizes RNA base hydrolysis. The presence of trace amounts of RNase can compromise RNA integrity, even if the samples are stored frozen in an aqueous environment. For short-term storage, RNA samples can be resuspended in RNase-free water (with 0.1 mM EDTA) or TE buffer (10 mM Tris, 1 mM EDTA) and stored at -80°C. Using a buffer solution that contains a chelating agent is a better way to store RNA. Chelation of divalent cations such as Mg2+ and Ca2+ will prevent heat-induced strand scission (RNA can be chemically cleaved when heated in the presence of Mg2+). Therefore, the combination of the oral wash and stabilizer formulations described herein are also stabilizing for RNA after collection, during sample prep for analysis, during sample storage (room temp 25°C, low temp 4°C, -20°C, or -80°C), or sample shipment where temperatures can be elevated above room typical room temperatures (>25°C).

Methods for detecting SARS-CoV-2 RNA in a sample are known in the art, for example, using real time PCR.

The CDC 2019-Novel Coronavirus (2019-nCoV) Real-Time RT-PCR Diagnostic Panel is a real-time RT-PCR test intended for the qualitative detection of nucleic acid from the 2019-nCoV in upper and lower respiratory specimens. The oligonucleotide primers and probes for detection of 2019-nCoV were selected from regions of the virus nucleocapsid (N) gene. The panel is designed for specific detection of the 2019-nCoV (two primer/probe sets). An additional primer/probe set to detect the human RNase P gene (RP) in control samples and clinical specimens is also included in the panel. In brief, RNA isolated and purified from a collected specimen is reverse transcribed to cDNA and subsequently amplified in the Applied Biosystems 7500 Fast Dx Real-Time PCR Instrument with SDS version 1.4 software. In the process, the probe anneals to a specific target sequence located between the forward and reverse primers. During the extension phase of the PCR cycle, the 5' nuclease activity of Taq polymerase degrades the probe, causing the reporter dye to separate from the quencher dye, generating a fluorescent signal. With each cycle, additional reporter dye molecules are cleaved from their respective probes, increasing the fluorescence intensity. Fluorescence intensity is monitored at each PCR cycle by Applied Biosystems 7500 Fast Dx Real-Time PCR System with SDS version 1.4 software.

Total nucleic acid can be extracted from collected saliva samples using methods known in the art. For example, total RNA can be extracted from about 300 µl of whole saliva using the MagMAX Viral/Pathogen Nucleic Acid Isolation kit (ThermoFisher Scientific) following the manufacturer's protocol and eluted into 75 µl of elution buffer.

For SARS-CoV-2 RNA detection, 5 µl of RNA template was tested as previously described using the US CDC real-time RT-PCR primer/probe sets for 2019-nCoV_N1 and 2019-nCoV_N2 and the human RNase P (RP) as an extraction control. Samples were classified as positive for SARS-CoV-2 when both N1 and N2 primer-probe sets were detected <38 C*T*. (Wylie, et al., doi: https://doi.org/10.1101/2020.04.16.20067835). RT-PCR kits for SARS-CoV-2 detection are readily available. The Real-Time PCR kits are based on widely used nucleic acid amplification technology. The products contain oligonucleotide primers, labeled oligonucleotide probes, and control material used in real-time RT-PCR for the in vitro qualitative detection of SARS-CoV-2 RNA in respiratory specimens. Exemplary kits include the *Quick* SARS-CoV-2 rRT-PCR Kit (Zymo Reseasrch); the RayBio^{®} Coronavirus (SARS-CoV-2) Real Time RT-PCR Nucleic Acid Detection Kit (Catalog #: PCR-COV). The Centers for Disease Control and Prevention (CDC) has developed an RT-PCR assay for SARS-COv-2, known as the CDC 2019-Novel Coronavirus Real-time Reverse Transcriptase (RT)_PCR diagnostic Panel.

RT-PCR can be a qualitative (yes/no) result, or it can be quantitative (qPCR (Frootan, et al., Methods to determine limit of detection and limit of quantification in quantitative real-time PCT (qPCR)", Biomolecular Detection and Quantification 12 (2017) 1-6).

The disclosed compositions and methods can be used to collect saliva samples for the ultimate determination of the presence of immunoglobulins (Ig), such as IgM, IgA or IgG, against a particular pathogen, and a conclusion of prior exposure to the pathogen. When a pathogen such as a virus invades host, the body produces large amounts of immunoglobulin (Ig) by the immune system and released into blood, among them, IgG, IgM, and IgA isotypes. It is widely believed that IgM is the first antibody to be transiently synthesized in response to the virus invasion. 15 IgG is a major class of immunoglobulins found in the blood, comprising 75% of total serum immunoglobulins and has long-term immunity and immunological memory. Various studies have correlated antibody levels in blood to levels in saliva. IgG profiles in plasma and saliva are highly similar for each individual. As a consequence, one could implement practically any plasma-based IgG assay (classical serology) as saliva based assay. Ma, et al., serum IgA level positively correlated with COVID-19 disease severity (doi: https://doi.org/10.1101/2020.04.17.20064907). IgG can be isolated from saliva samples, collected according to the methods disclosed herein, as disclosed for example, in Hettegger, et al., PLoS ONE 14(6): e0218456. (2019). In short, saliva is centrifuged for removing heavy components, microfiltrated to remove particles and large molecules and subsequently buffer exchanged with binding buffer for purification by Protein G affinity chromatography.

Saliva samples, collected according to the methods disclosed herein can therefore serve as a source for IgG, IgM, and IgA isotypes, and these Ig's can be identified is the sample adapting immunoassays developed for SARS-CoV-2 IgG, IgM, and IgA, disclosed for example in Ma, et al., dot:https://doi.org/10.1101/2020.04.17.20064907, or the Cellex qSARS-CoV-2 IgG/IgM Rapid Test. The Cellex qSARS-CoV-2 IgG/IgM Rapid Test is a lateral flow chromatographic immunoassay which can detect antibodies against the SARS-CoV-2 virus. The test cassette consists of 1) a burgundy colored conjugate pad containing SARS-CoV-2 recombinant antigens conjugated with colloidal gold (SARS-CoV-2 conjugates) and rabbit IgG-gold conjugates; 2) a nitrocellulose membrane strip containing an IgG line (G Line) coated with anti-human IgG, an IgM line (M Line) coated with anti-human IgM, and the control line (C Line) coated with goat anti-rabbit IgG. When a correct volume of test specimen is dispensed into the sample well of the test cassette, the specimen migrates by capillary action along the cassette. The anti-SARS-CoV-2 virus IgG, if present in the specimen, will bind to the SARS-CoV-2 conjugates. If IgG is present in the specimen, the immunocomplex will then captured by the anti-human IgG line, forming a burgundy colored G Line, indicating a SARS-CoV-2 virus IgG positive test result. The anti-SARS-CoV-2 virus IgM, if present in the specimen, will bind to the SARS-CoV-2 conjugates. The immunocomplex is then captured by the anti-human IgM line, forming a burgundy colored M Line, indicating a SARS-CoV-2 virus IgM positive test result. Information regarding the immune response to SARS-CoV-2 is limited and still evolving.

### B. Cancer Diagnostics

The disclosed oral wash can be used for cancer diagnostics. Compared to conventional cancer diagnostic methods, the use of oral samples is non-invasive and/or can allow for detection of a range of cancer-related biomarkers after a one-time sample collection. In some embodiments, the method involves detection of a single cancer-related biomarker. In some embodiments, the method involves detection of a panel of cancer-related biomarkers.

In some embodiments, the oral wash and/or the Post collection stabilization composition contains an internal tracer, for example a heavy isotope-labeled variant of the analyte as an internal tracer. In some embodiments, the oral wash contains heavy isotope-labeled caffeine as an internal tracer. In some embodiments, the oral wash does not contain any internal tracer. In a preferred embodiment, the internal tracer is present in the oral wash.

Salivary biomarkers for cancer are generally known in the art. See, for example, Ngamchuea et al., Analyst, 2018, 143, 81-99; Castagnola et al., Acta Otorhinolaryngologica Italica, 2017;37:94-101; AI-Tarawneh et al., OMICS, 2011, 15(6):353-61; and Saxena et al., Adv Biomed Res. 2017; 6: 90.

The cancer-related biomarkers include small molecules such as nitrates and nitrites, uric acid, valine, lactic acid, phenylalanine, propionylcholine, N-acetyl-L-phenylalanine, sphinganine, phytosphingosine and S-carboxymethyl-L-cysteine, or a combination thereof.

The cancer-related biomarkers also include large molecules such as proteins and nucleic acids (DNA and RNA, especially mRNA). Exemplary protein and nucleic acid-based salivary biomarkers are shown in Tables 3-5. Additional protein-based salivary biomarkers include interleukins (such as interleukins 6, 8 and 1b), cyclin D1 thioredoxin, profiling 1, resistin, thrombospondin-2, S100A8, α1-antitrypsin (AAT), haptoglobin β chains (HAP), complement C3, 4B, factor B, leucine-rich α-2-glycoprotein, galectine-7, 2-macroglobulin, ceruloplasmin, cystatin B, triose-phosphate isomerase and a protein called "deleted in malignant tumor 1 protein", keratin 10, and haemopexin and transthyretin.

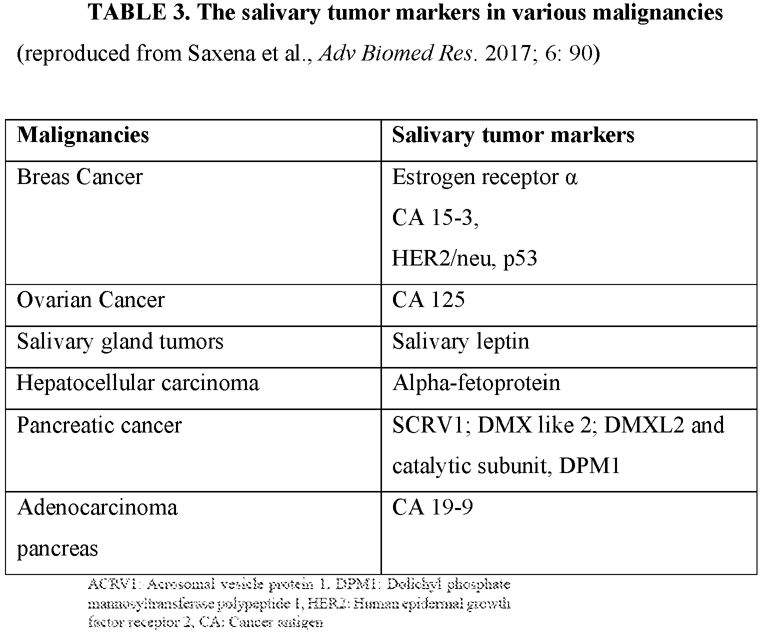

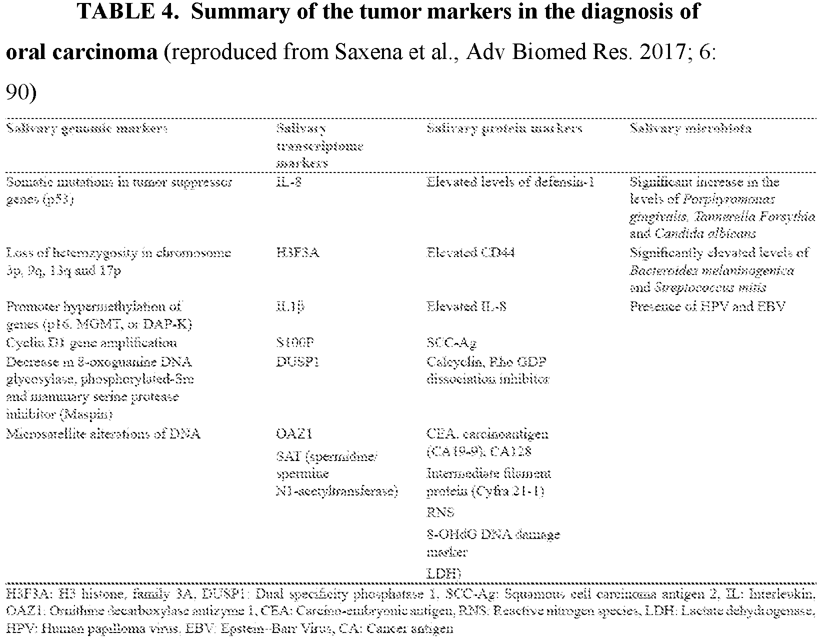

**TABLE 5. Exemplary nucleic acid-based biomarkers for genetic diagnosis of oral squamous cell carcinoma (reproduced from Markopoulos et al., The Open Dentistry Journal, 2010, 4, 172-178)**

| **Changes in the Cellular DNA** | **Altered mRNA transcript** | **Altered protein** |
|---|---|---|
| Allelic loss on chromosome 9p | Presence of IL8 | Elevated levels of defensin-1 |
| Mitochondrial DNA mutations | Presence of IL1B | Elevated CD44 |
| P53 gene mutations | DUSP1 (dual specificity phosphatase 1) | Elevated IL-6 and IL-8 |
| Promoter hypermethylation of genes (p16, MGMT or DAP-K) | H3F3A (H3 histone, family 3A) | Inhibitors of apoptosis (IAP) |
| Cyclin D1 gene amplification | OAZ1 (ornithine decarboxylase antizyme 1) | Squamous cell carcinoma associated antigen (SCC-Ag) |
| Increase of Ki67 markers | S100P (S100 calcium binding protein P0 | Carcino-embryonic antigen (CEA) |
| Microsatellite alterations of DNA | SAT (spermidine/spermine N1-acetyltransferase) | Carcino antigen (CA19-9) |
| Presence of HPV | | CA128 |
| | | Serum tumor marker (CA125) |
| | | Intermediate filament protein (Cyfra 21-1) |
| | | Tissue polypeptide specific antigen (TPS) |
| | | Reactive nitrogen species (RNS) |
| | | 8-OhdG DNA damage marker |
| | | Lactate dehydrogenase (LDH) |
| | | Immunoglobulin (IgG) |
| | | s-IgA |
| | | Insulin growth factor (IGF) |
| | | Metalloproteinase MMP-2 and MMP-11 |

The oral wash can be used to detect, diagnose or monitor different types of cancer, including but not limited to, oral cavity cancer, gastric cancer, breast cancer, ovarian cancer, salivary gland tumor, hepatocellular carcinoma, pancreatic cancer, and adenocarcinoma pancreas. In preferred embodiments, the oral wash can be used to detect, diagnose or monitor oral cavity cancer such as oral squamous cell carcinoma.

### C. Detection of Drugs and Drug Metabolites

The oral wash can be used to detect drugs and drug metabolites. Compared to blood-based detection methods, the use of oral samples is non-invasive and/or can allow for quick, safe, and easy sample collection, especially for in-field applications. Compared to urine-based detection methods, the use of oral samples can provide more accurate time-related information on drug consumption.

In some embodiments, the oral wash contains a heavy isotope-labeled variant of the analyte as an internal tracer. In some embodiments, the oral wash contains heavy isotope-labeled caffeine as an internal tracer. In some embodiments, the oral wash does not contain any internal tracer.

### Opioids

Exemplary opioids that can be detected using the quantitative pointof-care assay include morphine, codeine, thebaine, heroin, hydromorphone, hydrocodone, oxycodone, oxymorphone, desomorphine, nicomorphine, propoxyphene, dipropanoylmorphine, benzylmorphine, ethylmorphine, buprenorphine, fentanyl, pethidine, meperidine, methadone, tramadol, dextropropoxyphene, or analogues or derivatives thereof. For example, oxycodone (OxyContin^{®}) is an opioid analgesic medication synthesized from opium-derived thebaine. Percocet is a combination of oxycodone and acetaminophen (paracetamol). Vicodin is a combination of hydrocodone and acetaminophen (paracetamol). In preferred embodiments, the assay quantitatively measures oxycodone, hydrocodone, or a combination thereof.

Exemplary opioid metabolites that can be detected using the disclosed quantitative lateral flow immunoassay are shown in Table 6.

**Table 6. Opioid metabolites**

| Opioid | Key metabolizing enzyme(s) | Major metabolites |
|---|---|---|
| Buprenorphine | CYP3A4 | Norbuprenorphine, glucuronides |
| Codeine | CYP3A4, 2D6 | Morphine, glucuronides |
| Fentanyl | CYP3A4 | Norfentanyl |
| Hydrocodone | CYP3A4, 2D6 | Hydromorphone, norhydrocodone |
| Hydromorphone | UGT1A3, 2B7 | Glucuronides |
| Meperidine | CYP3A4, 2B6, 2C19 | Normeperidine |
| Methadone | CYP2B6 | EDDP |
| Morphine | UGT2B7 | Glucuronides |
| Oxycodone | CYP3A4, 2D6 | Noroxycodone, oxymorphone |
| Oxymorphone | UGT2B7 | 6-OH-oxymorphone, oxymorphone-3-glucuronide |
| Propoxyphene | CYP3A4 | Norpropoxyphene |
| Tramadol | CYP2D6 | O-desmethyl tramadol |

| | | |
|---|---|---|
| EDP = 2-Ethyl-1,5-dimethyl-3,3-diphenylpyrrolinium. | | |

### Marijuana and cannabinoids

In the cannabis plant, THC occurs mainly as tetrahydrocannabinol carboxylic acid (THC-COOH). Geranyl pyrophosphate and olivetolic acid react, catalyzed by an enzyme to produce cannabigerolic acid, which is cyclized by the enzyme THC acid synthase to give THC-COOH. Over time, or when heated, THC-COOH is decarboxylated producing THC. THC is metabolized mainly to 11-OH-THC (11-hydroxy-THC, denoted as HTHC) by the human body. This metabolite is still psychoactive and is further oxidized to 11-Nor-9-carboxy-THC (THC-COOH). More than 100 metabolites n humans and animals can be identified, but 11-OH-THC and THC-COOH are the dominating metabolites. Metabolism occurs mainly in the liver by cytochrome P450 enzymes CYP2C9, CYP2C19, and CYP3A4. More than 55% of THC is excreted in the feces and approximately 20% in the urine. The main metabolite in urine is the ester of glucuronic acid and THC-COOH and free THC-COOH. In the feces, mainly 11-OH-THC is detected.

THC, 11-OH-THC (HTHC), and THC-COOH can be detected and quantified in blood, urine, hair, oral fluid or sweat. The concentrations obtained from such analyses can often be helpful in distinguishing active from passive use or prescription from illicit use, the route of administration (oral versus smoking), elapsed time since use and extent or duration of use.

### Lithium

Lithium compounds, also known as lithium salts, are primarily used as a psychiatric medication. This includes the treatment of major depressive disorder that does not improve following the use of other antidepressants, and bipolar disorder. Lithium compounds are generally taken by mouth. Exemplary lithium compounds include, but are not limited to, lithium carbonate, lithium citrate, lithium orotate, lithium bromide, lithium chloride, lithium fluoride, and lithium iodide.

### Nicotine

As nicotine enters the body, it is distributed quickly through the bloodstream and crosses the blood-brain barrier reaching the brain within 10-20 seconds after inhalation. The elimination half-life of nicotine in the body is around two hours. The amount of nicotine absorbed by the body from smoking depends on many factors, including the types of tobacco, whether the smoke is inhaled, and whether a filter is used. For chewing tobacco, dipping tobacco, snus and snuff, which are held in the mouth between the lip and gum, or taken in the nose, the amount released into the body tends to be much greater than smoked tobacco.

Nicotine is metabolized in the liver by cytochrome P450 enzymes (mostly CYP2A6, and also by CYP2B6). A major metabolite of nicotine that is excreted in the urine is cotinine, which is a reliable and necessary indicator of nicotine usage. Other primary metabolites include nicotine N'-oxide, nornicotine, nicotine isomethonium ion, 2-hydroxynicotine and nicotine glucuronide. Glucuronidation and oxidative metabolism of nicotine to cotinine are both inhibited by menthol, an additive to mentholated cigarettes, thus increasing the half-life of nicotine *in vivo.*

Nicotine (cotinine) can be quantified in blood, plasma, or urine to confirm a diagnosis of poisoning or to facilitate a suspected nicotine overdose related death investigation. Urinary or salivary cotinine concentrations are frequently measured for the purposes of pre-employment and health insurance medical screening programs. Careful interpretation of results is important, since passive exposure to cigarette smoke can result in significant accumulation of nicotine, followed by the appearance of its metabolites in various body fluids.

The CYP2A6 enzyme is genetically polymorphic with certain alleles predicting altered metabolic activity. As the primary enzyme for nicotine metabolism, variation in the metabolic activity of CYP2A6 has a significant effect on an individual's level of tobacco consumption. The reduced metabolism phenotype leads to higher blood/nicotine levels and smokers tend to compensate for this by smoking less. Conversely, individuals with increased metabolic rate tend to smoke more. Lower nicotine metabolism with CYP2A6 variants also has an effect on smoking cessation, with slow metabolizers demonstrating higher levels of cessation in transdermal nicotine therapy trials. This may be due to the higher therapeutic doses of nicotine that the slow metabolizer sub-group obtains from comparable levels of transdermal nicotine treatment. Normal metabolizers have lower cessation rates probably as a result of current treatments failing to provide high enough levels of replacement blood nicotine. These normal metabolizers may be candidates for higher-dose nicotine replacement, which might potentially give rise to adverse effects in those with impaired nicotine metabolism.

The disclosed compositions and methods may be used to evaluate a patient's metabolism of nicotine. For example, nicotine levels can be quantified using the disclosed compositions and methods after a controlled dosage of nicotine is administered to a patient. This can in some embodiments involve allowing the subject to smoke a cigarette. In preferred embodiments, a nicotine patch or gum is given to the subject for a prescribed amount of time. The amount of nicotine or a metabolite thereof (e.g., cotinine) in a biological sample of the subject may then be monitored for rate of change.

### Stimulants

Stimulants (also often referred to as psychostimulants or colloquially as uppers) is an overarching term that covers many drugs including those that increase activity of the central nervous system and the body, drugs that are pleasurable and invigorating, or drugs that have sympathomimetic effects. Stimulants are widely used throughout the world as prescription medicines as well as without a prescription (either legally or illicitly) as performanceenhancing or recreational drugs.

Amphetamines are a class of stimulants based on the amphetamine structure. They include all derivative compounds which are formed by replacing, or substituting, one or more hydrogen atoms in the amphetamine core structure with substituents. Examples of substituted amphetamines includes amphetamine (itself), methamphetamine, ephedrine, cathinone, phentermine, mephentermine, bupropion, methoxyphenamine, selegiline, amfepramone, pyrovalerone, MDMA (ecstasy), and DOM (STP). Many drugs in this class work primarily by activating trace amine-associated receptor 1 (TAAR1); in turn, this causes reuptake inhibition and effluxion, or release, of dopamine, norepinephrine, and serotonin. An additional mechanism of some amphetamines is the release of vesicular stores of monoamine neurotransmitters through VMAT2, thereby increasing the concentration of these neurotransmitters in the cytosol, or intracellular fluid, of the presynaptic neuron.

Cocaine and its analogues are another class of stimulants. They usually maintain a benzyloxy connected to the carbon 3 of a tropane. Various modifications include substitutions on the benzene ring, as well as additions or substitutions in place of the normal carboxylate on the tropane 2 carbon. Various compounds with similar structure-activity relationships to cocaine that are not technically analogues have been developed as well. Exemplary cocaine analogues include stereoisomers of cocaine, 3β-phenyl ring substituted analogues, 2β-substituted analogues, N-modified analogues of cocaine, 3β-carbamoyl analogues, 3β-alkyl-3-benzyl tropanes, 6/7-substituted cocaines, 6-alkyl-3-benzyl tropanes, and piperidine homologues of cocaine. Examples of cocaine analogues can be found in Singh, Chemistry, Design, and Structure-Activity Relationship of Cocaine Antagonists, Chem. Rev., 2000, 100, 925-1024.

### Central Nerve System Depressants

Central nerve system (CNS) depressants typically slow brain activity, which makes them useful for treating anxiety and sleep problems.

Common CNS depressants include barbiturates such as pentobarbital (NEMBUTAL^{®}), benzodiazepines, and sleep medications such as eszopiclone (LUNESTA^{®}), zaleplon (SONATA^{®}), and zolpidem (AMBIEN^{®}).

Benzodiazepines (BZD, BDZ, BZs), sometimes called "benzos", are a class of psychoactive drugs whose core chemical structure is the fusion of a benzene ring and a diazepine ring. Benzodiazepines enhance the effect of the neurotransmitter gamma-aminobutyric acid (GABA) at the GABA_{A} receptor, resulting in sedative, hypnotic (sleep-inducing), anxiolytic (anti-anxiety), anticonvulsant, and muscle relaxant properties. High doses of many shorteracting benzodiazepines may also cause anterograde amnesia and dissociation. These properties make benzodiazepines useful in treating anxiety, insomnia, agitation, seizures, muscle spasms, alcohol withdrawal and as a premedication for medical or dental procedures. Exemplary benzodiazepines include brotizolam, midazolam, triazolam, alprazolam, estazolam, flunitrazepam, clonazepam, lormetazepam, lorazepam, nitrazepam, temazepam, diazepam, clorazepate, chlordiazepoxide, flurazepam, halazepam, prazepam, oxazepam, nimetazepam, adinazolam, climazolam, loprazolam, and derivatives thereof.

### Other Drugs and Drug Metabolites

Hallucinogens are psychoactive agents, which can cause hallucinations, perceptual anomalies, and other substantial subjective changes in thoughts, emotion, and consciousness. The common types of hallucinogens are psychedelics, dissociatives, and deliriants. Although hallucinations are a common symptom of amphetamine psychosis, amphetamines are not considered hallucinogens, as they are not a primary effect of the drugs themselves. Exemplary hallucinogens include ketamine, LSD (Lysergic acid diethylamide) and other ergotamine derivatives, mescaline and other phenethylamines, PCP (Phencyclidine), psilocybin, salvia, DMT and other tryptamines, and ayahuasca. Psychedelics include serotonergics and cannabinoidergics. Serotonergics can be further divided into indoles/tryptamines (such as psilocybin, ergolines such as LSD, betacarbolines, and complexly substituted tryptamines such as ibogaine) and phenethylamines such as mescaline.

γ-Hydroxybutyric acid (GHB), also known as 4-hydroxybutanoic acid, is a naturally occurring neurotransmitter and a psychoactive drug. It is a precursor to GABA, glutamate, and glycine in certain brain areas. It acts on the GHB receptor and is a weak agonist at the GABA_{B} receptor. Its prodrugs and analogs include 3-methyl-GHB, 4-methyl-GHB, 4-phenyl-GHB, 4-hydroxy-4-methylpentanoic acid (UMB68), γ-valerolactone (GVL), 1,4-butanediol diacetate (BDDA/DABD), methyl-4-acetoxybutanoate (MAB), ethyl-4-acetoxybutanoate (EAB), and γ-hydroxybutyraldehyde (GHBAL).

Additional drugs include mitragynine, 7-hydroxymitragynine, and derivatives thereof.

Additional drugs also include steroids such as nandrolone (OXANDRIN^{®}), oxandrolone (ANADROL^{®}), oxymetholone (ANADROL-50^{®}), and testosterone cypionate (DEPO-TESTOSTERONE^{®}).

Additional drugs also include Methylphenidate, ethylphenidate, and ritalinic acid. Methylphenidate is a stimulant medication used to treat attention deficit hyperactivity disorder (ADHD) and narcolepsy. Ethylphenidate acts as both a dopamine reuptake inhibitor and norepinephrine reuptake inhibitor, meaning it effectively boosts the levels of the norepinephrine and dopamine neurotransmitters in the brain, by binding to, and partially blocking the transporter proteins that normally remove those monoamines from the synaptic cleft. Ritalinic acid is a substituted phenethylamine and an inactive major metabolite of the psychostimulant drugs methylphenidate and ethylphenidate.

Aripiprazole is an atypical antipsychotic. It is primarily used in the treatment of schizophrenia and bipolar disorder. Other uses include as an add-on treatment in major depressive disorder, tic disorders and irritability associated with autism. A metabolite of aripiprazole, OPC-3373, is much more water soluble and can partition to oral fluids better than the parent compound and a more commonly assayed metabolite dehydro aripiprazole. All of the foregoing three compounds can be the analytes.

Additional metabolites that can be assayed from a saliva sample collected using the disclosed oral wash and methods include 10-11-Dihydro-10-Hydroxycarbamazepine, 2-Hydroxyethylflurazepam, 4-Methylephedrine, 6a-Naloxol, 6b-Naltrexol, 6-MAC, 6-MAM, 7-Aminoclonazepam, 7-Hydroxyquetiapine, 9-Hydroxyrisperidone, Alfentanil, Amitriptyline, Amo-Pentobarbital, Benzoylecgonine, Butabarbital, Butalbital, Caffeine, Cannabidiol, Carbamazepine, Citalopram, Cocaethylene, Cyclobenzaprine, Desalkylflurazepam, Desipramine, Desmethylcitalopram, Desmethylcyclobenzaprine, Desmethyldoxepin, Desmethyltapentadol, Dextromethorphan, Dextrorphan, Dihydrocodeine, Doxepin, Fluoxetine, Gabapentin, Hydroxyalprazolam, Hydroxymidazolam, Hydroxytriazolam, Imipramine, mCPP (meta-Chlorophenylpiperazine), MDA (3,4-Methylenedioxyamphetamine), MDEA (3,4-Methylenedioxy-N-ethylamphetamine), MDMA (3,4-Methylenedioxymethamphetamine), commonly known as ecstasy, MDPV (Methylenedioxypyrovalerone), Methylone, Nalbuphine, Naloxone, Naltrexone, Nordiazepam, Norketamine, Nortriptyline, O-Desmethyl-cis-Tramadol, O-Desmethylvenlafaxine, Oxcarbazepine, Paroxetine, Pentazocine, Phenobarbital, Pregabalin, Primidone, Protriptyline, Quetiapine, Risperidone, Secobarbital, Sertraline, Sufentanil, Tapentadol, Topiramate, Trazodone, Venlafaxine, Zaleplon, and Zolpidem-P4CA.

### D. Alcohol Detection

When coupled with either using direct injection gas chromatography (GC) or headspace gas chromatography (HS-GC) with flame ionization detection (FID) and/or mass spectrometry (GC-MS), the oral wash can be used for alcohol detection. Compared to conventional alcohol breathometers, which may only provide presumptive results, the use of oral samples generates much more robust testing results that can be directly correlated with standard blood tests. Further, the use of oral samples can allow for in-field tests that will eliminate unnecessary time delay. In some embodiments, the oral wash contains ¹³C and/or ²H-labeled alcohol as an internal tracer. In some embodiments, the oral wash contains heavy isotope-labeled caffeine as an internal tracer. In some embodiments, the oral wash does not contain any internal tracer.

The disclosed methods will be understood by the following non-limiting examples.

### Example 1: The impact of pH pre and post collection on absorptive loss of analyte in a saliva sample as a function of collection container material

In this example, ~25 mM Citric acid was used. The citric acid was titrated with solid NaOH then then mixed to make the same solution ratios used for the calibrators in the oral HPLC-MS method which is 2 mL of Oral wash + 1 mL artificial saliva (SOW). In this case the citric acid buffer solutions were compared to the current wash formulation at pH 6.0 as this formulation also includes EDTA, Allura Red dye, Sucralose, and flavoring esters. HiQC (High Quality Control, 25 ng/mL for all analytes stock solution into 3 mL aliquots of these SOW solutions at the different pHs into a 50 mL Protein LoBind container from Eppendorf. 1 mL of the same solutions was aliquoted into amber glass autosampler vials, spiked with HiQC stock (1: 100 dilution), Internal Standards (ISTD) were added (deuterated for HPLC-MS analysis), and then aliquoted into Thomson filter vials. In this way the influence of pH modulated adsorptive losses to plastics can be separated into losses due to the collection vial versus the Thomson filter vial used during the lab prep.

After spiking HiQC stock and allowing to stand at room temp for several hours, 1 mL was pipetted into glass amber glass autosampler vials and 300 uL ISTD stock was added and vortexed. This solution was tested on the HPLC-MS method without further processing (no filter vial).

The 1 mL of aliquoted SOW at the different pHs was spiked with HiQC stock (1:100 dilution), 300 uL ISTD stock was added, vortexed and then 450 uL of this solution was added to Thomson filter vials and pressed for analysis on the HPLC-MS.

The full set of results are included in a table format below across the whole panel and shown below, in relevant part. A ratio of over 1 indicates increased adsorptive losses at higher pH

**Table 7. Influence of simulated collected oral fluid pH on adsorptive losses in plastic collection container**

| **#** | **Name** | **Col_4.5** | **Col_6.0** | **Col_8.0** | **Col_9.1** | **Col_Orig_6.0** | **ratio 4.5/9.1** |
|---|---|---|---|---|---|---|---|
| 108 | THC | 14.4 | 13.4 | 0.4 | 0.5 | 0.5 | 27.94 |
| 23 | Cannabidiol | 14.4 | 12.9 | 2.3 | 2.3 | 2.7 | 5.43 |
| 109 | THC-COOH | 23.9 | 28.3 | 18.3 | 15.6 | 5.8 | 4.11 |
| 110 | THC-OH | 15.5 | 12.9 | 3.8 | 3.1 | 4.1 | 3.81 |
| 33 | Dehydro Aripiprazole | 23.6 | 20.0 | 2.2 | 2.2 | 11.8 | 2.00 |
| 16 | Aripiprazole | 25.8 | 22.4 | 2.9 | 2.8 | 14.8 | 1.74 |

The "Col" refers to the collector prep.

The ratio below shows not much difference across the pH range when the samples are only exposed to glass.

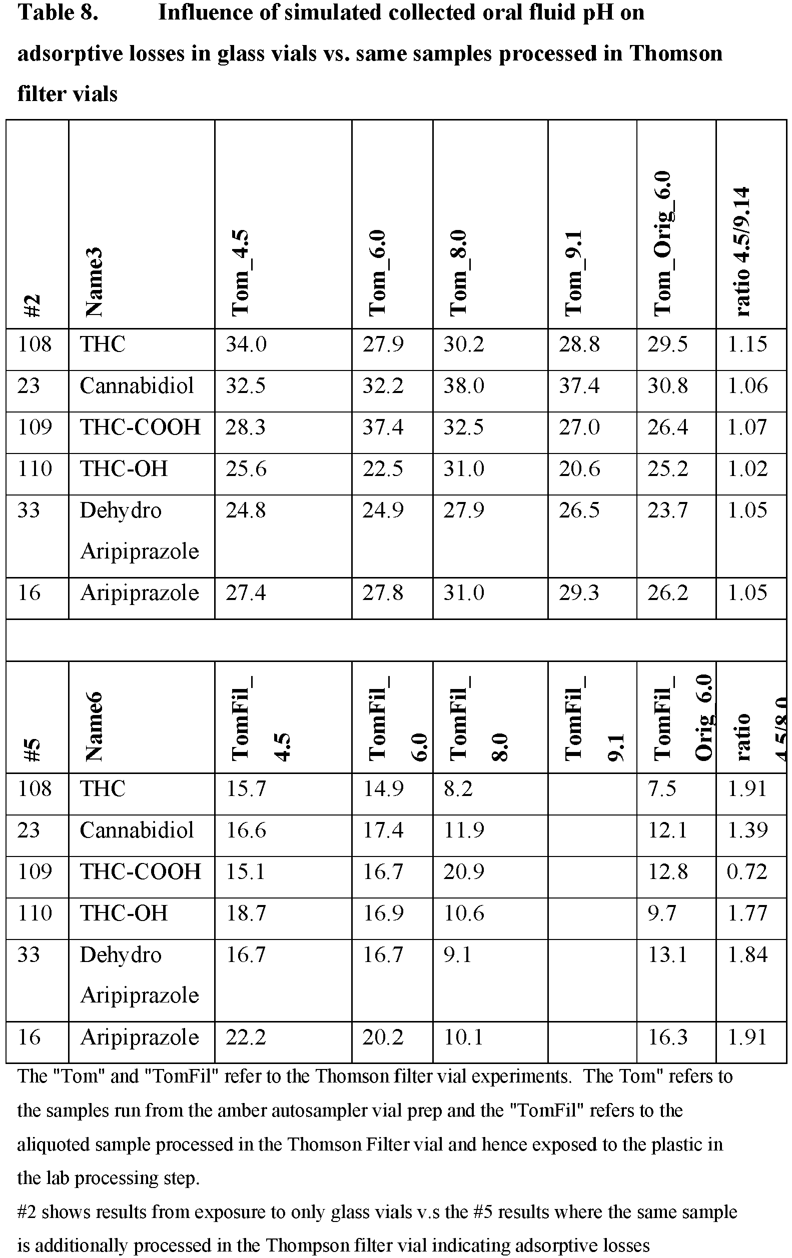

There are also clear examples of analytes that are better at high pH in the Thomson Filter vials. This trend was not clear on the collectors.

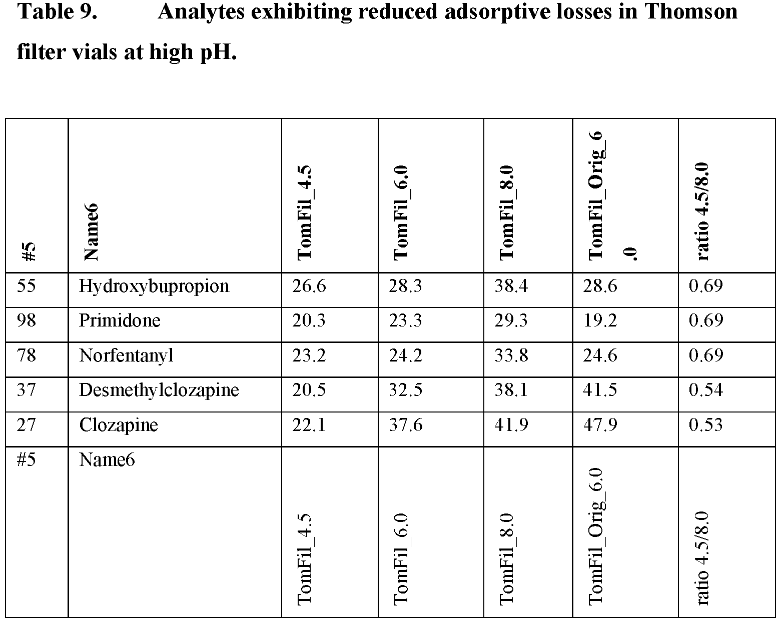

These studies also confirmed that LoBind is better than regular Polypropylene (PP) 50 mL just looking at post collection addition of ~100 mg of Citric acid. LoBind or similar plastics (hydrophilic surface) are the preferred plastics for the collector. In Table 4, various processing combinations of regular PP vs. LoBind, with or without added citrate, and glass sample vial vs. Thomson filter vial are shown comparing the residual THC detected. Since THC was the most problematic analyte, with regard to adsorptive losses observed in this study, the data clearly indicates that LoBind type plastics are preferrable for the initial collection container to reduce adsorptive losses. While glass is also indicated to be preferrable for post collection analysis, the necessity of sample filtration prior to HPLC-MS analysis and addition of matched ISTDs (THC-D3 for this analyte) to ameliorate losses is shown to produce comparable results to glass with or without the addition of citrate lowering the final pH to the desired range for sample storage stability.

**Table 10. Detected concentrations of THC analyte (25 ng/mL) under various process conditions**

| **Sample Name** | **Conc. (ng/ml)** |
|---|---|
| Regular Glass | 0.6 |
| Regular Glass | 0.7 |
| Regular+Citrate Glass | 0.1 |
| Regular+Citrate Glass | 0.0 |
| LoBind Glass | 11.9 |
| LoBind Glass | 11.9 |
| LoBind+Citrate Glass | 12.7 |
| LoBind+Citrate Glass | 11.4 |
| Regular Filter Vial | 2.3 |
| Regular Filter Vial | 2.8 |
| Regular+Citrate Filter Vial | 1.1 |
| Regular+Citrate Filter Vial | 1.1 |
| LoBind Filter Vial | 13.1 |
| LoBind Filter Vial | 12.9 |
| LoBind+Citrate Filter Vial | 13.1 |

### Example 2. Effect Oral wash composition on RNA

The phosphodiester bond of RNA is most stable at pH 4-5 at 90°C. RNA is susceptible to alkaline hydrolysis at pH > 6, whereas, in contrast, acid hydrolysis only occurs at pH < 2. Nerhhardt, et al., *Biology Direct* 2012, 7:4. Studies were conducted to determine the effect of oral wash pH and components on RNA.

4 oral wash solutions prepared (referred to as: basic with dye, basic without dye, acidic with dye, acidic without dye), and synthetic saliva added to the oral wash. 75uL of each solution was placed into a separate 1.5mL tube then inoculated with 75uL of Quantitative Synthetic SARSCoV2 RNA: ORF, E, N (ATCC^{®} VR3276SD ^{™}) RNA. The resulting solution had a concentration of 1000 copies per uL. Samples were left in 4 degrees Celsius for 72 hours.

After 72 hour incubation at 4 degrees Celsius additional samples were prepared. 100 uL of each solution above inoculated with 100uL of ATCC RNA. The resulting solution had a concentration of 1000 copies per uL. (The samples prepared to 150uL were brought up to 200uL using additional solution, this brought the estimated concentration to 750uL.). 1 nasopharyngeal swab was taken and placed into a 0.9% sterile saline solution as a control. Another 1 nasopharyngeal swab was taken and placed into a 0.45% sterile saline solution as a separate control.

Samples were extracted using Thermofisher viral pathogen kit and extractions were performed to extract virus on the Kingfisher Flex platform.

Samples were then run in a 384 well plate using Ray Biotech reagents and primer/probes.

Results were as follows, shown in Table 11.

**Table 11**

| **Sample name and concentration** | **N1 CT** | **N2 CT** |
|---|---|---|
| 72hr basic w/ dye (750 copies/µL) | 26.466 | 29.001 |
| 72hr basic w/o dye (750 copies/µL) | 27.309 | 30.516 |
| 72hr acidic w/ dye (750 copies/µL) | 26.344 | 28.894 |
| 72hr acidic w/o dye (750 copies/µL) | 26.350 | 28.851 |
| same day basic w/ dye (1000 copies/uL) | 23.566 | 26.090 |
| same day basic w/o dye (1000 copies/uL) | 23.820 | 26.301 |
| same day acidic w/ dye (1000 copies/uL) | 22.402 | 24.755 |
| same day acidic w/o dye (1000 copies/uL) | 22.222 | 24.675 |

The data show that: the fluid does retain RNA for at least 72 hours. Dye does not impact the detection of the probes in solution. Therefore, the disclosed oral wash solution can be used to collect and subsequently analyze samples for the presence of RNA, specifically, SARA-CoV-2 RNA. Not only does this address supply shortages on the nasopharyngeal swabs but the experience will be much easier, less painful than the current procedures (nasopharyngeal swabs/blood draw) and offer additional testing opportunities

### Example 3. Stability of SARS-CoV-2 RNA in oral rinse at room temperature

The purpose of this pilot project was to accomplish a proof of concept to demonstrate the effectiveness of the disclosed rinse solution (QuikSal^{™}) at stabilization of viral RNA, SARS-CoV-2 purified RNA from ATCC/BEI, at room temperature for at least a 72-hour period. **Procedure:** Eight 1mL aliquots of Oral Rinse Solution with and without SOW were made up. Two of the aliquots had 200,000 copies of the SARSCoV-2 Positive control (Integrated DNA Technologies) the other six aliquots were spiked to 20,000 copies per milliliter. The 200K and first set of 20K aliquots were immediately processed via the Zymo Quick-DNA/RNA Viral MagBead kit. The other aliquots were allowed to sit on the benchtop at room temperature for 24, 48 and 72 hours respectively, then processed via the Zymo Quick-DNA/RNA Viral MagBead kit, the samples were analyzed using the PathogenDx DetectX-Rv. The data is shown in Figure 1. No signal was picked up from the no template control rinse. **Conclusions:** SOW with or without dye is capable of stabilizing spiked viral RNA for up to 72 hours at room temperature.

Additionally, antigen binding studies post collection of SARS-CoV-2 are consistent with a conclusion that the virus is deactivated and therefore, rendered not infectious, when collected as disclosed herein.

### Example 4. Detection of SARS-CoV-2 using RT-PCR, in samples collected using oral wash and post collection stabilization solution

The following compositions were used in this experiment. New formulation (50 mM Citric Acid pH 4.2)

### 1. Oral Wash (1 L, 50 mM, pH 4.2)

| | |
|---|---|
| Trisodium Citrate dihydrate (mw: 294.1 g/mol): | 6.75 g |
| Citric Acid monohydrate (mw: 210.14 g/mol): | 5.65 g |
| Tartrazine: | 5 mg |

| | |
|---|---|
| Sucralose: | 100 mg |
| EDTA | 300 mg |
| Sodium Benzoate: | 500 mg |
| Potassium sorbate: | 500 mg |
| Butyl 2-methylbutyrate: | 6 uL |
| Methyl isobutyrate: | 6 uL |

### 2. Post Collection Stabilization Solution (1L, 1 M, pH 3.5)

| | |
|---|---|
| Trisodium Citrate dihydrate (mw: 294.1 g/mol): | 86.67 g |
| Citric Acid monohydrate (mw: 210.14 g/mol): | 147. 96 g |
| Methyl Orange: | 150 mg |

These experiments were performed in the same manner as described for the oral wash formulations using the Oral Wash + Saliva (human negative collection) +citrate. However, in this case a post collection stabilization solution was added. Methyl Orange was also included and found not to influence results.

### Results and interpretation.

When the RT-PCR COVID-19 assay is run with the standard protocol but using spiked SARS-CoV-2 RNA controls in oral wash + post stabilization solution with a real negative human collection the following data was collected Table 1 which indicates detection, relative to controls, down to 31.25 copies/µL.

The table shows samples collected using an oral wash without dyes (-) and samples collected with oral wash containing dyes (+). Although RT-PCR can be a quantitative assay, in this clinical setting it was used as a qualitative (yes/no) detection for presence of SARS-CoV-2. So in both experiments, with and without dye, a ct<40 is reported as detected. Thus, although there were some indeterminate and/or negative values in some of the replicates, the experiments indicated little if any interference down to a Limit of detection of 31.25 copies/uL. However, if one takes the average signal over all replicates and the three targets one can see a slight impact of the dyes as the ratio of signals is less than 1.0 starting at 250 copies/uL (-dye/+dye) but this effect is small and doesn't impact the qualitative output of the clinical SARS-CoV-2assay at a limit of detection well below the clinical cutoff set at 200 copies/uL. So, in that sense this data indicates that the dye has no impact on clinically reportable results from saliva samples collected using the disclosed oral wash and post collection stabilizations solutions.

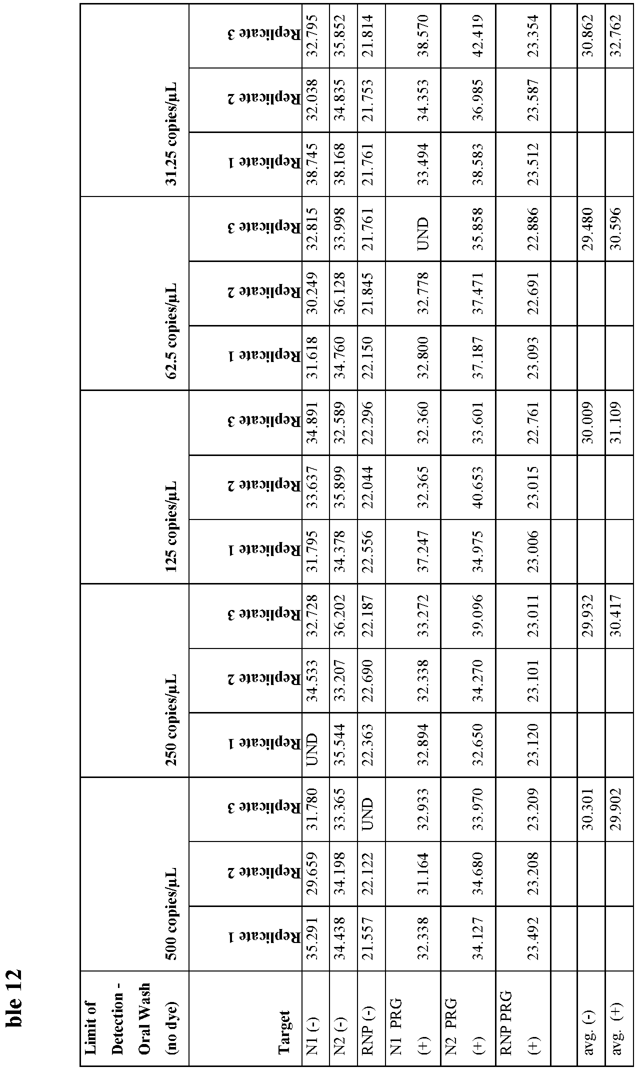

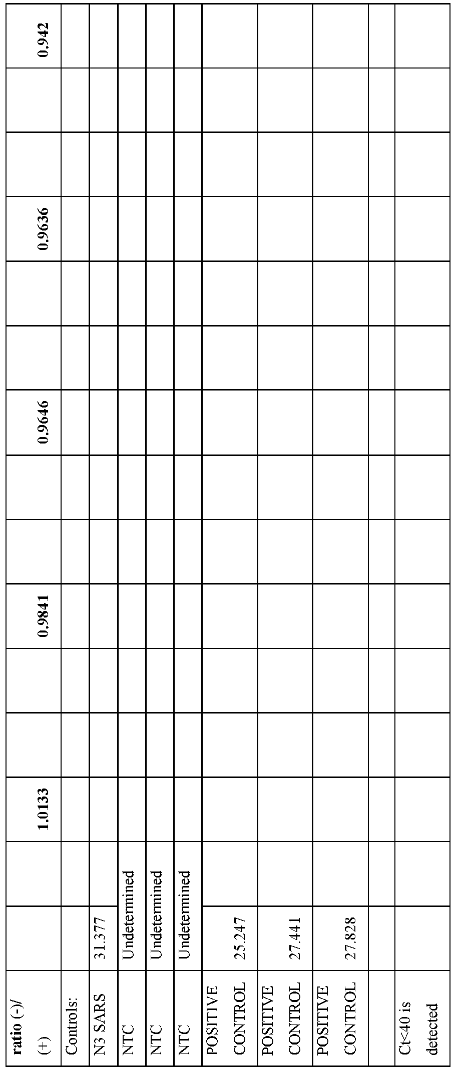

### Example 5. Comparison of ability to detect analyte using the disclosed oral wash, compared to analyte collected using Nasopharyngeal (NP) swabs

Nasopharyngeal (NP) swabs and oral rinse specimens were collected one after the other as paired samples from human subjects and results were generated for both specimen types using an EUA authorized gold standard assay. Nasopharyngeal swabs were run according to EUA-approved Thermo TaqPath COVID-19 Combo Kit procedure and are an approved specimen type. These results were used as the "gold standard" to compare the experimental oral wash results against. Patient consent was documented.

The data shows that sample collection using the disclosed oral wash results in analyte detection comparable to the gold standards as demonstrated herein using SARS-CoV-2.

The results are shown in the table below, including Ct values. Results are displayed by the analysis software as either "detected" or "not detected" without the end user performing any manual analysis.

Total results are summarized in the table below.

| **NP Result** | **Total Tested** | **Total Concordant w/Oral Rinse** | **Percent Concordance** |
|---|---|---|---|
| Detected | 30/30 | 14/14 | 100% |
| Not Detected | 30/30 | 21/21 | 100% |

In the second table, the MS2 target is an extraction and internal control (IC). The N gene, ORF1ab, and S gene targets are specific to SARS-CoV-2. The final result is a product of interpretation of listed Ct values by the Applied Biosystems COVID-19 Interpretive Software.

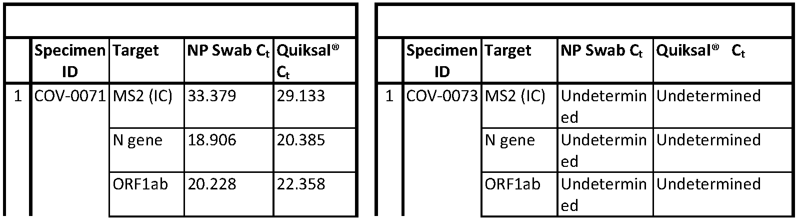

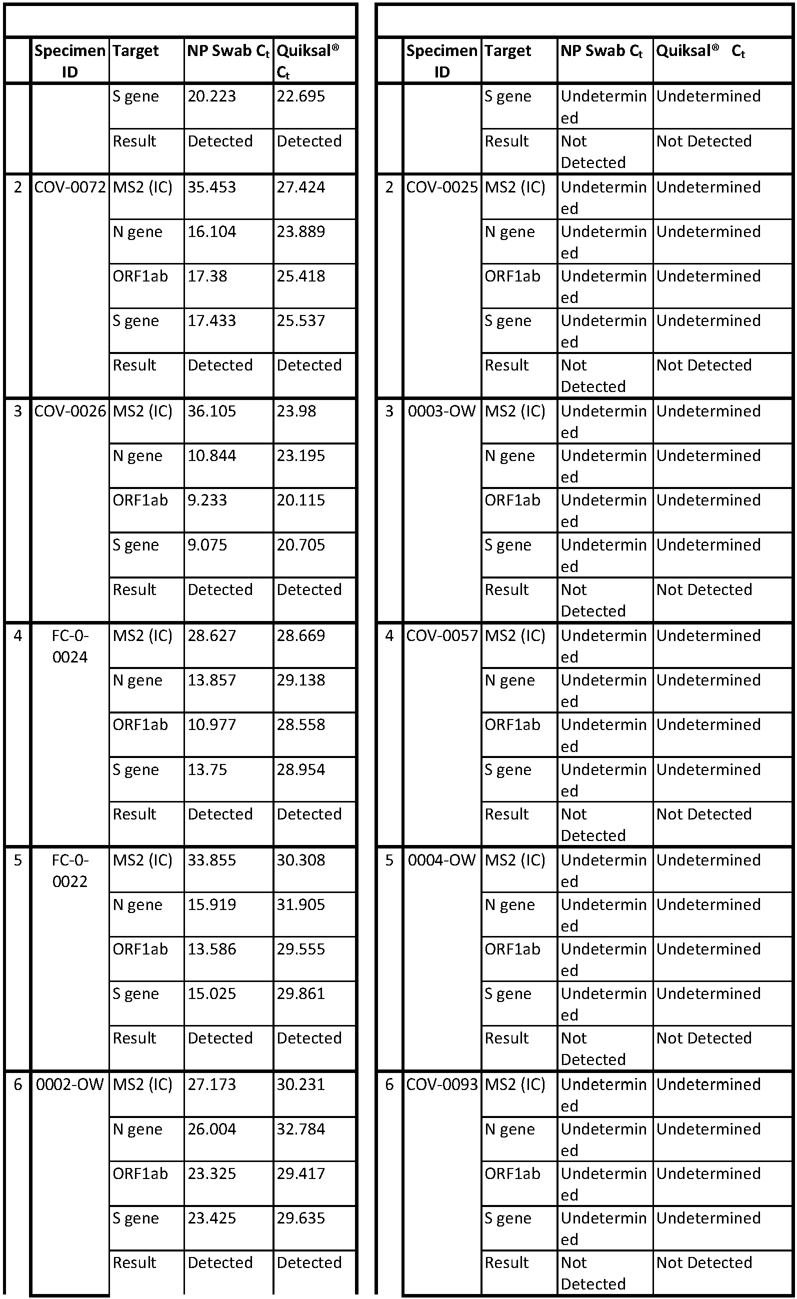

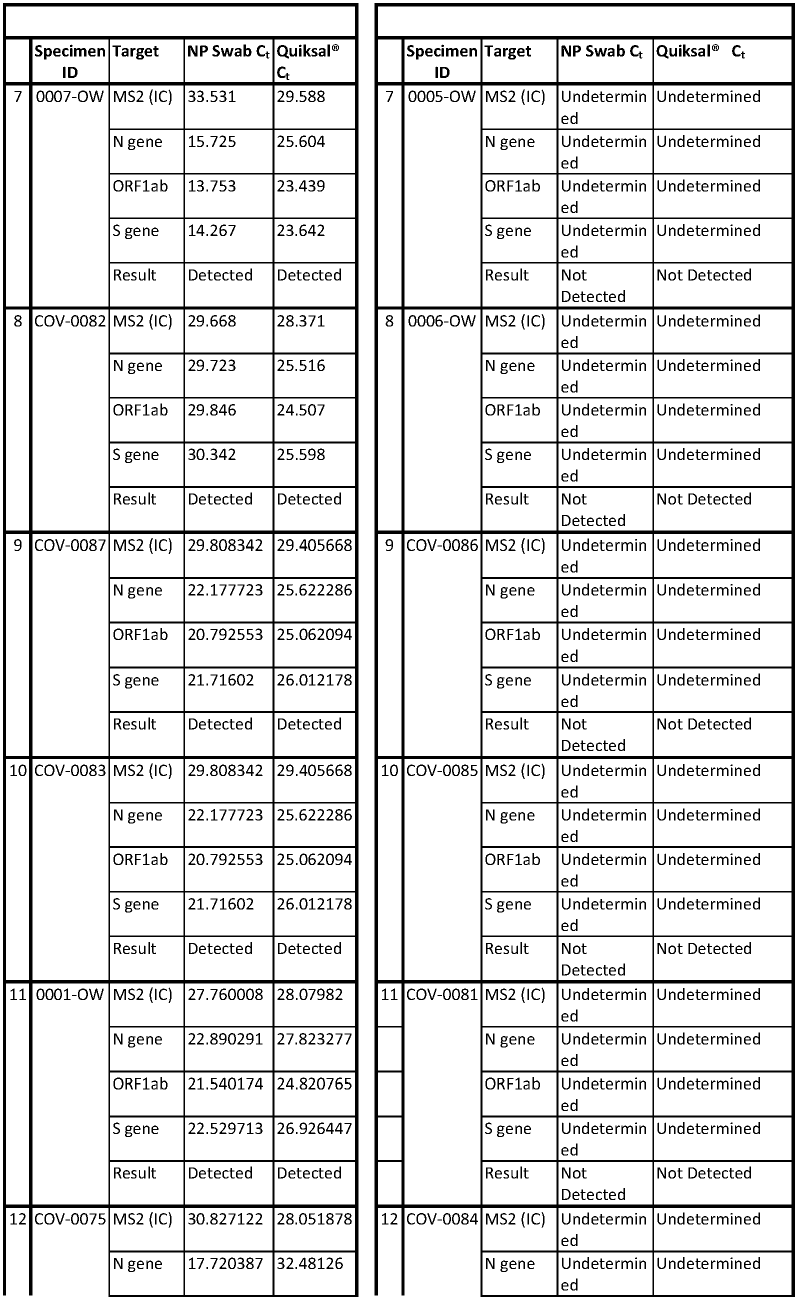

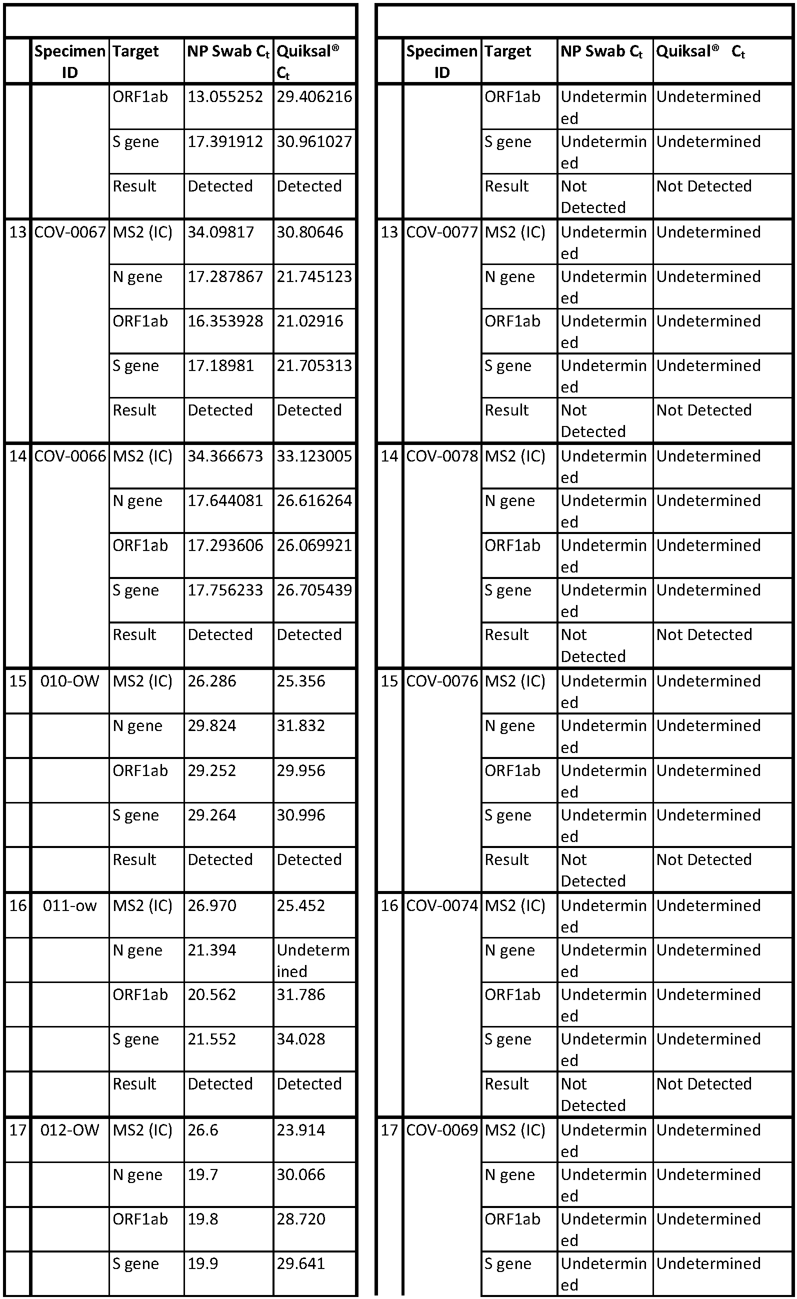

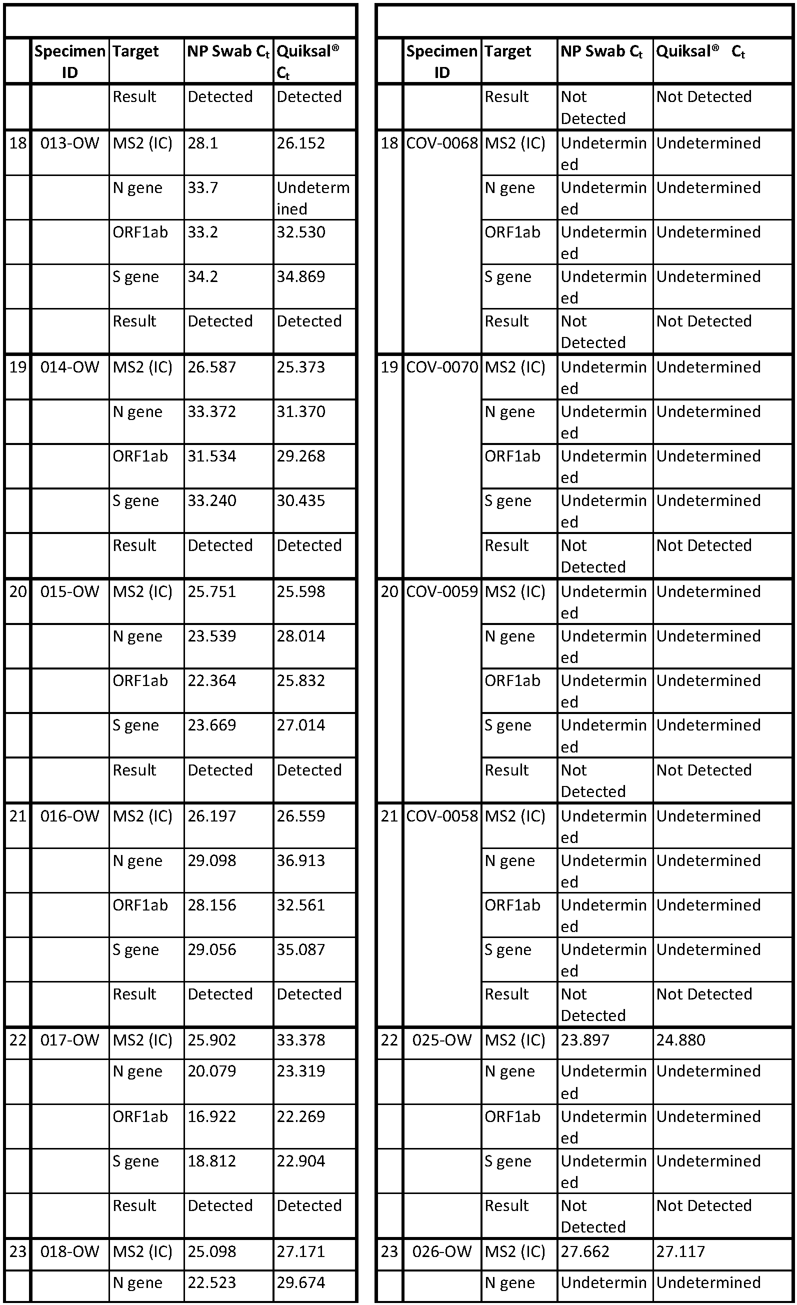

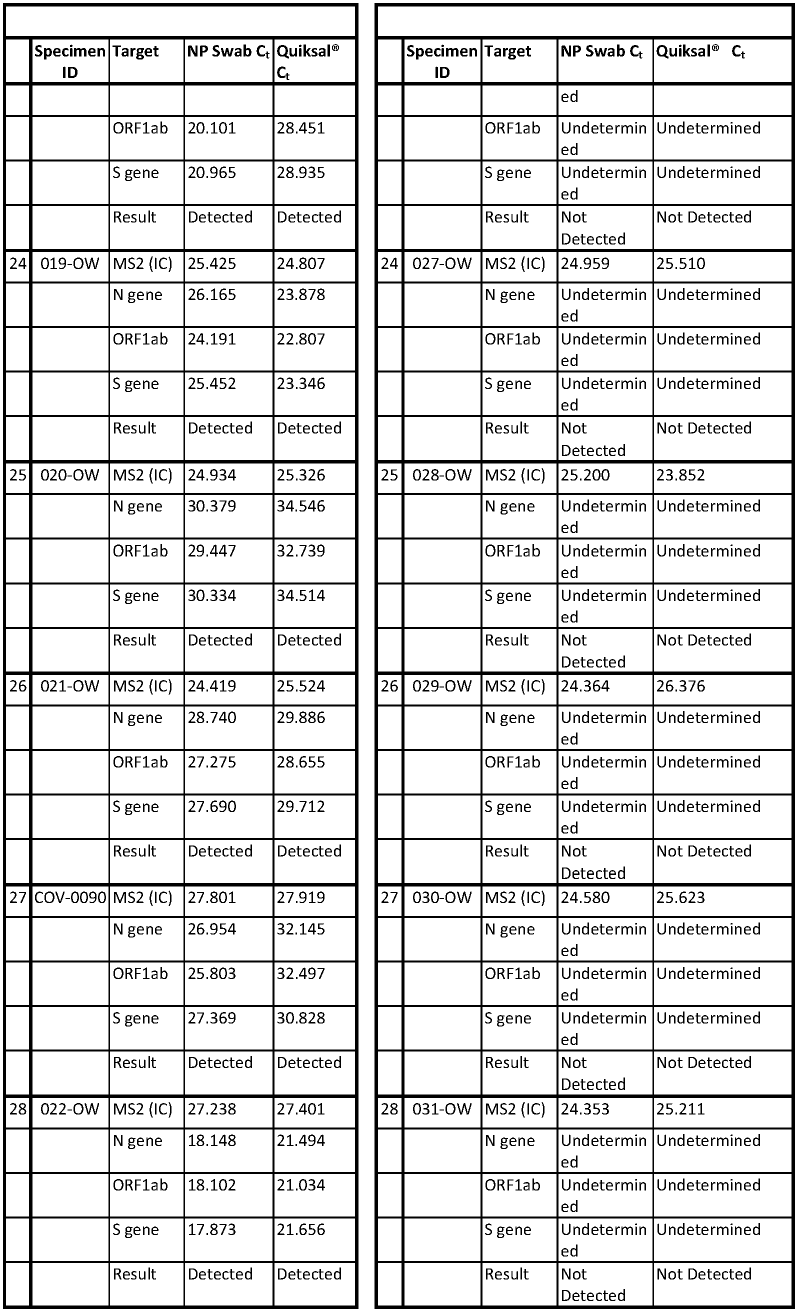

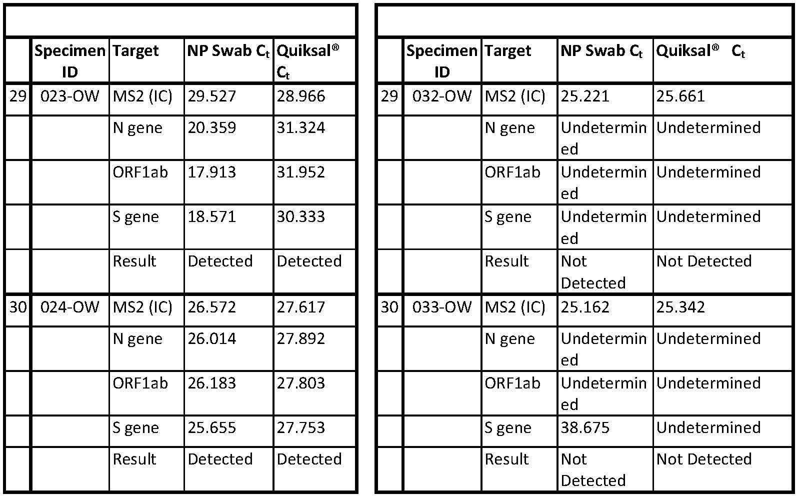

It must be noted that as used herein and in the appended claims, the singular forms "a," "an," and "the" include plural reference unless the context clearly dictates otherwise. Thus, for example, reference to "an antibody" includes a plurality of such antibodies, reference to "the antibody" is a reference to one or more antibodies and equivalents thereof known to those skilled in the art, and so forth.

Throughout the description and claims of this specification, the word "comprise" and variations of the word, such as "comprising" and "comprises," means "including but not limited to," and is not intended to exclude, for example, other additives, components, integers or steps.

"Optional" or "optionally" means that the subsequently described event, circumstance, or material may or may not occur or be present, and that the description includes instances where the event, circumstance, or material occurs or is present and instances where it does not occur or is not present.

Unless the context clearly indicates otherwise, use of the word "can" indicates an option or capability of the object or condition referred to. Generally, use of "can" in this way is meant to positively state the option or capability while also leaving open that the option or capability could be absent in other forms or embodiments of the object or condition referred to. Unless the context clearly indicates otherwise, use of the word "may" indicates an option or capability of the object or condition referred to. Generally, use of "may" in this way is meant to positively state the option or capability while also leaving open that the option or capability could be absent in other forms or embodiments of the object or condition referred to. Unless the context clearly indicates otherwise, use of "may" herein does not refer to an unknown or doubtful feature of an object or condition.

Ranges can be expressed herein as from "about" one particular value, and/or to "about" another particular value. When such a range is expressed, also specifically contemplated and considered disclosed is the range from the one particular value and/or to the other particular value unless the context specifically indicates otherwise. Similarly, when values are expressed as approximations, by use of the antecedent "about," it will be understood that the particular value forms another, specifically contemplated embodiment that should be considered disclosed unless the context specifically indicates otherwise. It will be further understood that the endpoints of each of the ranges are significant both in relation to the other endpoint, and independently of the other endpoint unless the context specifically indicates otherwise. It should be understood that all of the individual values and sub-ranges of values contained within an explicitly disclosed range are also specifically contemplated and should be considered disclosed unless the context specifically indicates otherwise. Finally, it should be understood that all ranges refer both to the recited range as a range and as a collection of individual numbers from and including the first endpoint to and including the second endpoint. In the latter case, it should be understood that any of the individual numbers can be selected as one form of the quantity, value, or feature to which the range refers. In this way, a range describes a set of numbers or values from and including the first endpoint to and including the second endpoint from which a single member of the set (i.e. a single number) can be selected as the quantity, value, or feature to which the range refers. The foregoing applies regardless of whether in particular cases some or all of these embodiments are explicitly disclosed.

## Claims

1. A kit comprising an oral wash composition and a post collection stabilization solution, wherein:
(a) the oral wash composition, comprises one or more salivating agents, one or more antimicrobial agents, and one or more dyes and
(b) the post collection stabilization composition comprises one or more antimicrobial agents and one or more dyes, wherein the post collection stabilization composition is buffered at a pH ranging between 3 and 6.

2. The kit of claim 1, wherein the oral wash composition further comprises an internal tracer suitable for detection by mass spectrometry.

3. The kit of claim 2, wherein:
(a) the internal tracer contains one or more heavy isotopes, preferably wherein the one or more heavy isotopes are selected from ¹³C, ¹⁵N, ²H, and combinations thereof, more preferably wherein the internal tracer is caffeine-¹³C₃; and/or
(b) the internal tracer is not tartrazine.

4. The kit of claim 1, wherein the oral wash composition comprises a yellow dye selected from the group consisting of tartrazine, sunset yellow and quinoline yellow.

5. The kit of any one of claims 1-4, further comprising flavor enhancing agents and sweetening agents.

6. The kit of any one of claims 1-5, wherein the oral wash composition has a pH between about 3.0 and about 6.2, preferably wherein the oral wash composition has a pH between about 3.0 and about 4.6.

7. The kit of any one of claims 1-6, wherein the oral wash composition comprises one or more agents selected from the group consisting of:
(a) Trisodium Citrate dihydrate in a concentration ranging from 3g/L to 10 g/L, preferably, between 5 g/L and 7 g/L, and more preferably, about 6.75 g/L;
(b) Citric Acid monohydrate, in a concentration ranging from 2 g/L - 9 g/L, preferably between 4 and 6g/L and more preferably, about 5.65 g/L;
(c) Sucralose in a concentration ranging from 50-200 mg/L, preferably between 80 and 150 mg/L, and more preferably, about 100 mg/L;
(d) Sodium Benzoate, in a concentration ranging from 100 to 1000 mg/L, preferably, between 300 and 700 mg/L, and more preferably, about 500 mg/L;
(e) Potassium sorbate in a concentration ranging from 100 to 1000 mg/L, preferably, between 300 and 700 mg/L, and more preferably, about 500 mg/L;
(f) Butyl 2-methylbutyrate in a concentration ranging from 1- 15 µL/L, preferably, between 3 and 10 µL/L and more preferably, about 6 µL/L; and
(g) Methyl isobutyrate 1-15 µL/L, preferably, between 3 and 10 µL/L and more preferably, about 6 µL/L in a concentration ranging from in a 1- 5 ml solution.

8. The kit of any one of claims 1-7, wherein the post collection stabilization composition has a pH between 3 and 4.

9. The kit of any one of claims 1-8, wherein the post collection stabilization composition comprises a red dye selected from the group consisting of ponceau 4R, carmoisine, erythrosine and allura red.

10. The kit of any one of claims 1-9, wherein the post collection stabilization composition comprises one or more agents selected from the group consisting of:
(a) Citric Acid monohydrate, in a concentration range between 40 and 500 g/L, preferably between 100 and 300 g/L, and more preferably, between 120 and 160 g/L; and
(b) Trisodium Citrate dihydrate in a concentration between 50 and 200 g/L, preferably between 70 and 150 g/L and more preferably, between 80 and 100 g/L in a 100 µl to 1 ml solution, preferably, as a 300-800 µL solution, and more preferably as a 500 µl solution.

11. A method for identifying an analyte in the saliva of a subject, comprising:
(a) rinsing the oral cavity of the subject with the oral wash composition as defined in any one of claims 1-7 for a period of time effective to stimulate saliva production;
(b) collecting a resulting oral fluid from step (a) into a container, wherein the oral fluid comprises the oral wash and the saliva of the subject; and
(c) contacting the oral fluid with the post collection stabilization composition as defined in any one of claims 1, and 8-10, and
(d) subjecting the oral fluid to an analytical technique selected from the group consisting of PCR, immunoassay and mass spectrometry instrumentation to detect the analyte.

12. The method of claim 11, wherein the analyte is a drug of abuse, optionally wherein the drug of abuse is selected from the group consisting of alcohol, cannabinoids, opioids, amphetamines, cocaine, benzodiazepines, and combinations thereof.

13. The method of claim 11, wherein:
(a) the analyte is a lithium compound, or
(b) the analyte is a biomarker for cancer, optionally wherein the biomarker is a protein or a nucleic acid.

14. The method of any one of claims 11-13, wherein the mass spectrometry instrumentation is LC-MS, GC-MS, CE-MS, HPLC-MS/MS, HPLC-TOF or MALDI-TOF, optionally wherein the method does not contain any step of photometric measurement.

15. The method of claim 13 or 14, wherein the analyte is RNA or an immunoglobulin (Ig) selected from the group consisting of IgA, IgG, and IgM, optionally wherein:
(a) the analyte is the SARS-CoV-2 RNA and the sample is subjected to RT-PCR to detect the presence of SARS-CoV-2 RNA, the method further comprising isolating total RNA from the sample, prior to RT-PCR; or
(b) the analyte is an anti-SARS-CoV-2 Ig, and the sample is subjected to an immunoassay to detect the presence of anti-SARS-CoV-2 in the sample.

## Patentansprüche

1. Kit, umfassend eine orale Waschzusammensetzung und eine Stabilisierungslösung nach dem Sammeln, wobei:
(a) die orale Waschzusammensetzung ein oder mehrere speichelbildende Mittel, ein oder mehrere antimikrobielle Mittel und einen oder mehrere Farbstoffe umfasst und
(b) die Stabilisierungszusammensetzung nach dem Sammeln ein oder mehrere antimikrobielle Mittel und einen oder mehrere Farbstoffe umfasst, wobei die Stabilisierungszusammensetzung nach dem Sammeln bei einem pH-Wert zwischen 3 und 6 gepuffert ist.

2. Kit nach Anspruch 1, wobei die orale Waschzusammensetzung ferner einen internen Tracer umfasst, der zum Erkennen durch Massenspektrometrie geeignet ist.

3. Kit nach Anspruch 2, wobei:
(a) der interne Tracer ein oder mehrere Schwerisotope enthält, vorzugsweise wobei das eine oder die mehreren Schwerisotope aus ¹³C, ¹⁵N, ²H und Kombinationen davon ausgewählt sind, stärker bevorzugt wobei der interne Tracer Koffein-¹³C₃ ist; und/oder
(b) der interne Tracer nicht Tartrazin ist.

4. Kit nach Anspruch 1, wobei die orale Waschzusammensetzung einen gelben Farbstoff umfasst, der aus der Gruppe ausgewählt ist, bestehend aus Tartrazin, Gelborange und Chinolingelb.

5. Das Kit nach einem der Ansprüche 1 bis 4, ferner umfassend geschmacksverstärkende Mittel und Süßungsmittel.

6. Kit nach einem der Ansprüche 1 bis 5, wobei die orale Waschzusammensetzung einen pH-Wert zwischen etwa 3,0 und etwa 6,2 aufweist, vorzugsweise wobei die orale Waschzusammensetzung einen pH-Wert zwischen etwa 3,0 und etwa 4,6 aufweist.

7. Kit nach einem der Ansprüche 1 bis 6, wobei die orale Waschzusammensetzung ein oder mehrere Mittel umfasst, die aus der Gruppe ausgewählt sind, bestehend aus:
(a) Trinatriumcitrat-Dihydrat in einer Konzentration zwischen 3 g/l und 10 g/l, vorzugsweise zwischen 5 g/l und 7 g/l, und noch bevorzugter etwa 6,75 g/l;
(b) Zitronensäure-Monohydrat in einer Konzentration zwischen 2 g/l-9 g/l, vorzugsweise zwischen 4 und 6 g/l und noch bevorzugter etwa 5,65 g/l;
(c) Sucralose in einer Konzentration zwischen 50 und 200 mg/l, vorzugsweise zwischen 80 und 150 mg/l, und noch bevorzugter etwa 100 mg/l;
(d) Natriumbenzoat in einer Konzentration zwischen 100 und 1000 mg/l, vorzugsweise zwischen 300 und 700 mg/l, und noch bevorzugter etwa 500 mg/l;
(e) Kaliumsorbat in einer Konzentration zwischen 100 und 1000 mg/l, vorzugsweise zwischen 300 und 700 mg/l, und noch bevorzugter etwa 500 mg/l;
(f) Butyl-2-methylbutyrat in einer Konzentration zwischen 1-15 µL/L, vorzugsweise zwischen 3 und 10 µL/L und noch bevorzugter etwa 6 µL/L; und
(g) Methylisobutyrat 1-15 µL/L. vorzugsweise zwischen 3 und 10 µL/L und noch bevorzugter etwa 6 µL/L in einer Konzentration zwischen 1-5 ml Lösung.

8. Kit nach einem der Ansprüche 1 bis 7, wobei die Stabilisierungszusammensetzung nach dem Sammeln einen pH-Wert zwischen 3 und 4 aufweist.

9. Kit nach einem der Ansprüche 1 bis 8, wobei die Stabilisierungszusammensetzung nach dem Sammeln einen roten Farbstoff umfasst, der aus der Gruppe ausgewählt ist, bestehend aus Ponceau 4R, Carmoisin, Erythrosin und Allurarot.

10. Kit nach einem der Ansprüche 1 bis 9, wobei die Stabilisierungszusammensetzung nach dem Sammeln ein oder mehrere Mittel umfasst, die aus der Gruppe ausgewählt sind, bestehend aus:
(a) Zitronensäure-Monohydrat in einem Konzentrationsbereich zwischen 40 und 500 g/l, vorzugsweise zwischen 100 und 300 g/l, und noch bevorzugter zwischen 120 und 160 g/l; und
(b) Trinatriumcitrat-Dihydrat in einer Konzentration zwischen 50 und 200 g/l, vorzugsweise zwischen 70 und 150 g/l und noch bevorzugter zwischen 80 und 100 g/l in einer Lösung von 100 µl bis 1 ml, vorzugsweise in einer Lösung von 300-800 µl und noch bevorzugter in einer Lösung von 500 µl.

11. Verfahren zum Identifizieren eines Analyten im Speichel eines Subjekts, umfassend:
(a) Spülen des Mundhohlraums des Subjekts mit der oralen Waschzusammensetzung, wie in einem der Ansprüche 1 bis 7 definiert, über einen Zeitraum, der wirksam ist, um die Speichelproduktion zu stimulieren;
(b) Sammeln eines resultierenden oralen Fluids aus Schritt (a) in einen Behälter, wobei das orale Fluid die orale Waschflüssigkeit und den Speichel des Subjekts umfasst; und
(c) Kontaktieren des oralen Fluids mit der Stabilisierungszusammensetzung nach dem Sammeln, wie in einem der Ansprüche 1 und 8 bis 10 definiert, und
(d) Aussetzen des oralen Fluids einer analytischen Technik, ausgewählt aus der Gruppe bestehend aus PCR-, Immunoassay- und Massenspektrometrieinstrumenten, um den Analyten zu erkennen.

12. Verfahren nach Anspruch 11, wobei der Analyt ein Medikament des Missbrauchs ist, optional wobei das Medikament des Missbrauchs aus der Gruppe ausgewählt ist, bestehend aus Alkohol, Cannabinoiden, Opioiden, Amphetaminen, Kokain, Benzodiazepinen und Kombinationen davon.

13. Verfahren nach Anspruch 11, wobei:
(a) der Analyt eine Lithiumverbindung ist, oder
(b) der Analyt ein Biomarker für Krebs ist, optional wobei der Biomarker ein Protein oder eine Nukleinsäure ist.

14. Verfahren nach einem der Ansprüche 11 bis 13, wobei das Massenspektrometrieinstrument LC-MS, GC-MS, CE-MS, HPLC-MS/MS, HPLC-TOF oder MALDI-TOF ist, wobei das Verfahren optional keinen Schritt der photometrischen Messung enthält.

15. Verfahren nach Anspruch 13 oder 14, wobei der Analyt RNA oder ein Immunglobulin (Ig) aus der Gruppe ausgewählt ist, bestehend aus IgA, IgG und IgM, optional wobei:
(a) der Analyt die SARS-CoV-2-RNA ist und die Probe einer RT-PCR ausgesetzt wird, um das Vorhandensein von SARS-CoV-2-RNA zu erkennen, das Verfahren ferner umfassend das Isolieren von Gesamt-RNA aus der Probe vor der RT-PCR; oder
(b) der Analyt ein Anti-SARS-CoV-2-Ig ist und die Probe einem Immunoassay ausgesetzt wird, um das Vorhandensein von Anti-SARS-CoV-2 in der Probe zu erkennen.

## Revendications

1. Kit comprenant une composition de bain de bouche et une solution de stabilisation post-collecte, dans lequel:
(a) la composition de bain de bouche comprend un ou plusieurs agents salivants, un ou plusieurs agents antimicrobiens et un ou plusieurs colorants et
(b) la composition de stabilisation post-collecte comprend un ou plusieurs agents antimicrobiens et un ou plusieurs colorants, dans lequel la composition de stabilisation post-collecte est tamponnée à un pH compris entre 3 et 6.

2. Kit selon la revendication 1, dans lequel la composition de bain de bouche comprend en outre un traceur interne adapté à la détection par spectrométrie de masse.

3. Kit selon la revendication 2, dans lequel:
(a) le traceur interne contient un ou plusieurs isotopes lourds, de préférence dans lequel l'un ou plusieurs isotopes lourds sont choisis parmi ¹³C, ¹⁵N, ²H et des combinaisons de ceux-ci, de préférence encore dans lequel le traceur interne est la caféine-¹³C₃ ; et/ou
(b) le traceur interne n'est pas la tartrazine.

4. Kit selon la revendication 1, dans lequel la composition de bain de bouche comprend un colorant jaune choisi dans le groupe constitué par la tartrazine, le jaune orangé et le jaune de quinoléine.

5. Kit selon l'une quelconque des revendications 1 à 4, comprenant en outre des agents améliorant le goût et des agents édulcorants.

6. Kit selon l'une quelconque des revendications 1 à 5, dans lequel la composition de bain de bouche a un pH compris entre environ 3,0 et environ 6,2, de préférence dans lequel la composition de bain de bouche a un pH compris entre environ 3,0 et environ 4,6.

7. Kit selon l'une quelconque des revendications 1 à 6, dans lequel la composition de bain de bouche comprend un ou plusieurs agents choisis dans le groupe constitué par :
(a) le citrate trisodique dihydraté dans une concentration allant de 3 g/l à 10 g/l, de préférence, entre 5 g/l et 7 g/l, et de manière davantage préférée, d'environ 6,75 g/l ;
(b) l'acide citrique monohydraté, dans une concentration allant de 2 g/l à 9 g/l, de préférence entre 4 et 6 g/l et de manière davantage préférée, d'environ 5,65 g/l ;
(c) le sucralose dans une concentration allant de 50 à 200 mg/l, de préférence entre 80 et 150 mg/l, et de manière davantage préférée, d'environ 100 mg/l ;
(d) le benzoate de sodium, dans une concentration allant de 100 à 1000 mg/l, de préférence, entre 300 et 700 mg/l, et de manière davantage préférée, d'environ 500 mg/l ;
(e) le sorbate de potassium à une concentration allant de 100 à 1000 mg/l, de préférence entre 300 et 700 mg/l, et de manière davantage préférée, d'environ 500 mg/l ;
(f) le 2-méthylbutyrate de butyle dans une concentration allan de 1 à 15 µl/l, de préférence entre 3 et 10 µl/l et de manière davantage préférée, d'environ 6 µl/l ; et
(g) l'isobutyrate de méthyle de 1 à 15 µl/l, de préférence, entre 3 et 10 µl/l et de manière davantage préférée, d'environ 6 µl/l dans une concentration allant de 1 à 5 ml de solution.

8. Kit selon l'une quelconque des revendications 1 à 7, dans lequel la composition de stabilisation post-collecte a un pH compris entre 3 et 4.

9. Kit selon l'une quelconque des revendications 1 à 8, dans lequel la composition de stabilisation post-collecte comprend un colorant rouge choisi dans le groupe constitué par le ponceau 4R, la carmoisine, l'érythrosine et le rouge allura.

10. Kit selon l'une quelconque des revendications 1 à 9, dans lequel la composition de stabilisation post-collecte comprend un ou plusieurs agents choisis dans le groupe constitué par :
(a) l'acide citrique monohydraté, dans une plage de concentration comprise entre 40 et 500 g/l, de préférence entre 100 et 300 g/l, et de manière davantage préférée, entre 120 et 160 g/l ; et
(b) le citrate trisodique dihydraté à une concentration comprise entre 50 et 200 g/l, de préférence entre 70 et 150 g/l et de manière davantage préférée, entre 80 et 100 g/l dans une solution de 100 µl à 1 ml, de préférence, sous forme de solution de 300 à 800 µl, et de manière davantage préférée sous forme de solution de 500 µl.

11. Procédé d'identification d'un analyte dans la salive d'un sujet, comprenant :
(a) le rinçage de la cavité buccale du sujet avec la composition de bain de bouche telle que définie dans l'une quelconque des revendications 1 à 7 pendant une période efficace pour stimuler la production de salive ;
(b) le recueil d'un fluide buccal résultant de l'étape (a) dans un récipient, dans lequel le fluide buccal comprend le bain de bouche et la salive du sujet ; et
(c) la mise en contact du fluide buccal avec la composition de stabilisation post-collecte telle que définie dans l'une quelconque des revendications 1 et 8 à 10, et
(d) la soumission du fluide buccal à une technique d'analyse choisie dans le groupe constitué par la PCR, l'immuno-essai et l'instrumentation de spectrométrie de masse pour détecter l'analyte.

12. Procédé selon la revendication 11, dans lequel l'analyte est une drogue, éventuellement dans lequel la drogue est choisie dans le groupe constitué par l'alcool, les cannabinoïdes, les opioïdes, les amphétamines, la cocaïne, les benzodiazépines et des combinaisons de ceux-ci.

13. Procédé selon la revendication 11, dans lequel :
(a) l'analyte est un composé de lithium, ou
(b) l'analyte est un biomarqueur du cancer, éventuellement dans lequel le biomarqueur est une protéine ou un acide nucléique.

14. Procédé selon l'une quelconque des revendications 11 à 13, dans lequel l'instrumentation de spectrométrie de masse est LC-MS, GC-MS, CE-MS, HPLC-MS/MS, HPLC-TOF ou MALDI-TOF, éventuellement dans lequel le procédé ne contient aucune étape de mesure photométrique.

15. Procédé selon la revendication 13 ou 14, dans lequel l'analyte est de l'ARN ou une immunoglobuline (Ig) choisie dans le groupe constitué par IgA, IgG et IgM, éventuellement dans lequel :
(a) l'analyte est l'ARN du SARS-CoV-2 et l'échantillon est soumis à une RT-PCR pour détecter la présence d'ARN du SARS-CoV-2, le procédé comprenant en outre l'isolement de l'ARN total de l'échantillon, avant la RT-PCR ; ou
(b) l'analyte est une Ig anti-SARS-CoV-2, et l'échantillon est soumis à un immuno-essai pour détecter la présence d'anti-SARS-CoV-2 dans l'échantillon.
